(19) Europäisches Patentamt
European Patent Office
Office européen des brevets

(11) Veröffentlichungsnummer : **0 033 156**
**B1**

(12) # EUROPÄISCHE PATENTSCHRIFT

(45) Veröffentlichungstag der Patentschrift : **17.10.84**

(21) Anmeldenummer : **81100576.8**

(22) Anmeldetag : **27.01.81**

(51) Int. Cl.³ : **C 07 C 91/30**, C 07 C 93/14, C 07 C103/38, C 07 C103/737, C 07 C125/065, C 07 C 43/225, C 07 D209/12, C 07 C 47/27, A 61 K 31/135// C07C121/70, C07C121/76, C07C121/75, C07C143/68, C07C69/734, C07C59/54

(54) **1-Phenyl-2-cyclohexen-1-alkylamin-Derivate, Verfahren zu deren Herstellung und diese enthaltende Arzneimittel.**

(30) Priorität : **29.01.80 CH 724/80**

(43) Veröffentlichungstag der Anmeldung : **05.08.81 Patentblatt 81/31**

(45) Bekanntmachung des Hinweises auf die Patenterteilung : **17.10.84 Patentblatt 84/42**

(84) Benannte Vertragsstaaten : **AT BE CH DE FR GB IT LI LU NL SE**

(56) Entgegenhaltungen :
**EP-A- 0 012 801**
**US-A- 3 564 100**
**CHEMICAL ABSTRACTS; Band 69, Nr. 21, 18. November 1968, Seite 8066, Nr. 86438h, Columbus, Ohio, U.S.A., G. SCHWENKER et al.: "Preparation of DL-1-aryl-1-(2-dimethylaminoethyl)-2-cyclohexenes"**
**TETRAHEDRON, Band 35, 1979, Seiten 255-257, Oxford, G.B., K.PSOTTA et al.: " The total synthesis of joubertinamine "**

(73) Patentinhaber : **F. HOFFMANN-LA ROCHE & CO. Aktiengesellschaft**
**CH-4002 Basel (CH)**

(72) Erfinder : **Bruderer, Hans, Dr.**
**Stegmatten 34**
**CH-4105 Benken (CH)**
Erfinder : **Fischli, Albert Eduard, Prof. Dr.**
**Am Ausserberg 20**
**CH-4125 Riehen (CH)**
Erfinder : **Pfister, Rudolf, Dr.**
**Neubadstrasse 128**
**CH-4054 Basel (CH)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

**EP 0 033 156 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

In der US-Patentschrift No 3 564 100 werden u. a. 1-Phenyl-1-cyclohexen-2-alkylamin-Derivate beschrieben, welche besonders ausgeprägte antitussive und gute analgetische, antiphlogistische und antiproliferative Eigenschaften besitzen.

Die vorliegende Erfindung betrifft 1-Phenyl-2-cyclohexen-1-alkylamin-Derivate der allgemeinen Formel

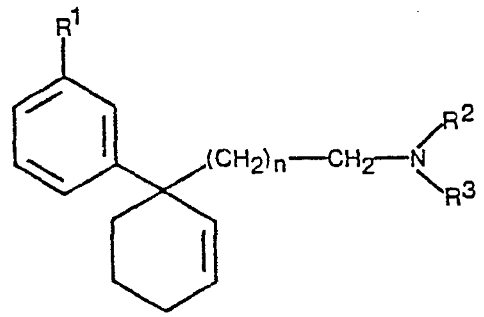

(I)

worin $R^1$ Hydroxy oder niederes Alkoxy, n die Zahl 1 oder 2, $R^2$ niederes Alkyl und $R^3$ Wasserstoff, niederes Alkyl, niederes Alkenyl oder niederes Cycloalkylmethyl bedeuten.

Diese Verbindungen sind neu und zeichnen sich durch wertvolle pharmakodynamische Eigenschaften aus.

Gegenstand der vorliegenden Erfindungen sind Verbindungen der allgemeinen Formel I und pharmazeutisch akzeptable Säureadditionssalze davon als solche und als pharmazeutische Wirkstoffe, die Herstellung dieser Verbindungen, ferner Arzneimittel, enthaltend eine Verbindung der allgemeinen Formel I oder ein pharmazeutisch akzeptables Säureadditionssalz davon, und die Herstellung solcher Arzneimittel.

Die Verbindungen der eingangs definierten allgemeinen Formel I enthalten ein asymmetrisches Kohlenstoffatom ; die vorliegende Erfindung umfasst sowohl die optisch einheitlichen enantiomeren Formen dieser Verbindungen als auch Gemische davon, insbesondere die Racemate.

Unter den Verbindungen der eingangs definierten Formel I sind diejenigen bevorzugt, worin $R^1$ Hydroxy oder Methoxy, $R^2$ und $R^3$ je niederes Alkyl (insbesondere je Methyl) und n die Zahl 1 bedeuten.

Eine besonders bevorzugte, von der allgemeinen Formel I umfasste Verbindung im Rahmen der vorliegenden Erfindung ist das 1-(m-Methoxyphenyl)-N,N-dimethyl-2-cyclohexen-1-äthylamin ; dies gilt sowohl für die beiden optisch einheitlichen enantiomeren Formen dieser Verbindung als auch für Gemische davon, insbesondere für das Racemat, welches im Rahmen der vorliegenden Erfindung ganz besonders bevorzugt ist.

Eine ebenfalls bevorzugte, von der allgemeinen Formel I umfasste Verbindung im Rahmen der vorliegenden Erfindung ist das 1-(m-Hydroxyphenyl)-N,N-dimethyl-2-cyclohexen-1-äthylamin.

Weitere repräsentative Vertreter der durch die allgemeine Formel I definierten Verbindungsklasse sind das 1-(m-Methoxyphenyl)-N,N-dimethyl-2-cyclohexen-1-propylamin, das 1-(m-Methoxyphenyl)-N-methyl-2-cyclohexen-1-äthylamin, das N-Allyl-1-(m-methoxyphenyl)-N-methyl-2-cyclohexen-1-äthylamin und das N-Cyclopropylmethyl-1-(m-methoxyphenyl)-N-methyl-2-cyclohexen-1-äthylamin.

Die Verbindungen der allgemeinen Formel I und deren pharmazeutisch akzeptable Säureadditionssalze können erfindungsgemäss dadurch hergestellt werden, dass man

a) eine Verbindung der allgemeinen Formel

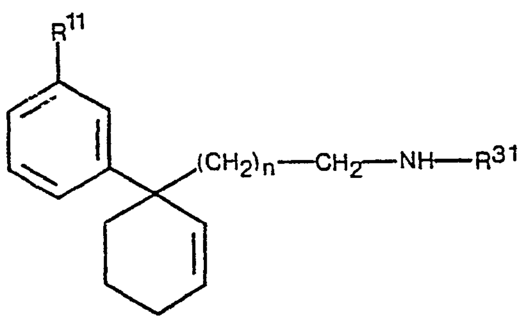

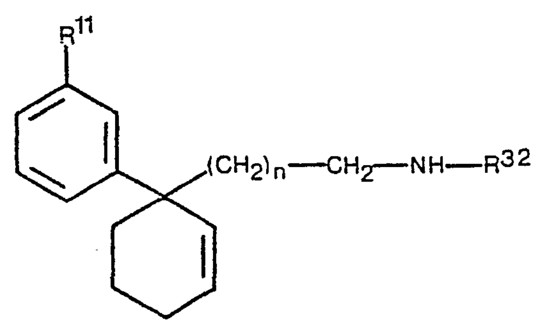

(II) oder (Ia)

worin n obige Bedeutung besitzt und $R^{11}$ niederes Alkoxy, $R^{31}$ Wasserstoff, niederes Alkenyl oder niederes Cycloalkylmethyl und $R^{32}$ niederes Alkyl bedeuten, am Stickstoffatom entsprechend substituiert

oder

b) eine Verbindung der allgemeinen Formel

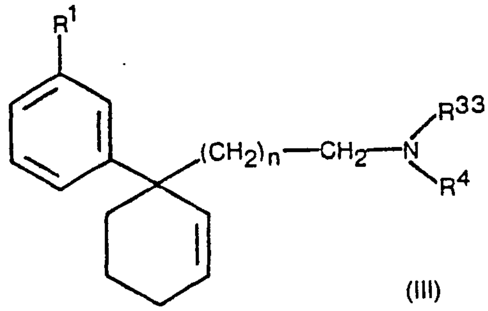

(III) oder

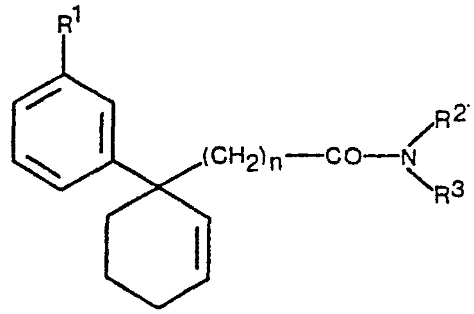

(IV)

worin $R^1$, $R^2$, $R^3$ und n obige Bedeutung besitzen und entweder $R^{33}$ Wasserstoff, niederes Alkyl, niederes Alkenyl oder niederes Cycloalkylmethyl und $R^4$ Formyl, niederes Alkanoyl oder niederes Alkoxycarbonyl oder $R^{33}$ niederes Alkyl und $R^4$ niederes Cycloalkylcarbonyl bedeuten, reduziert oder

c) eine Verbindung der allgemeinen Formel

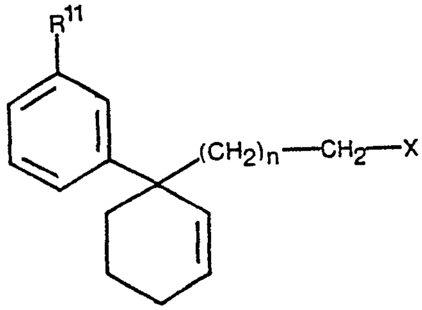

(V)

worin $R^{11}$ und n obige Bedeutung besitzen und X eine Abgangsgruppe bedeutet, mit einem Amin der allgemeinen Formel

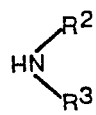

(VI)

worin $R^2$ und $R^3$ obige Bedeutung besitzen, umsetzt oder

d) eine quartäre Ammoniumbase der allgemeinen Formel

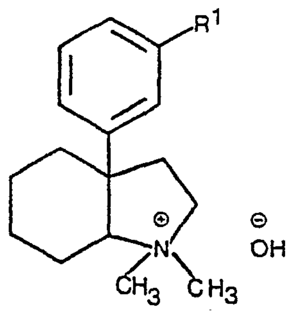

(VII)

worin $R^1$ obige Bedeutung besitzt, erhitzt oder ein geeignetes entsprechendes quartäres Ammoniumsalz erhitzt oder mit einer Base behandelt, oder

e) aus einer Verbindung der allgemeinen Formel

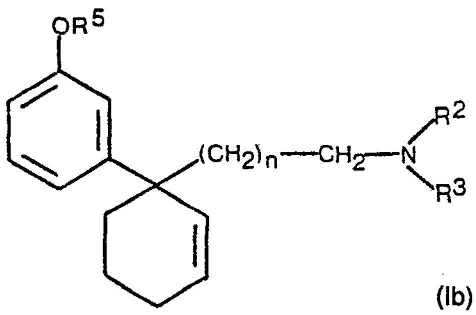

(Ib) oder

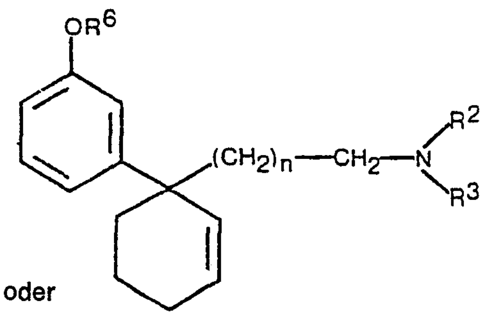

(VIII)

worin $R^2$, $R^3$ und n obige Bedeutung besitzen und $R^5$ niederes Alkyl und $R^6$ eine Schutzgruppe bedeuten, die Alkylgruppe $R^5$ bzw. die Schutzgruppe $R^6$ abspaltet oder

f) eine Verbindung der allgemeinen Formel

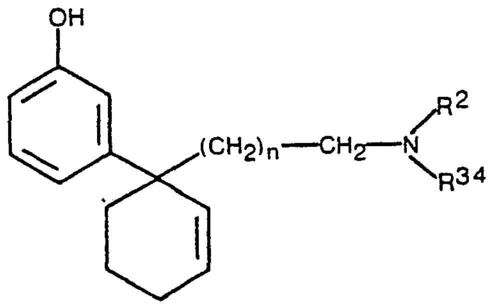

(Ic)

worin n und $R^2$ obige Bedeutung besitzen und $R^{34}$ niederes Alkyl, niederes Alkenyl oder niederes Cycloalkylmethyl bedeutet, veräthert oder

g) eine Verbindung der allgemeinen Formel

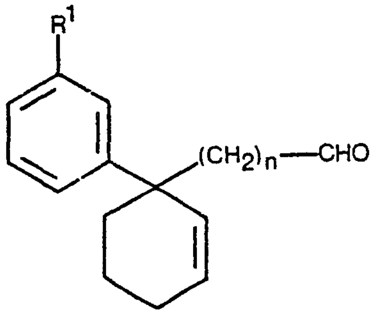

(IX)

worin $R^1$ und n obige Bedeutung besitzen, unter reduzierenden Bedingungen mit einem Amin der obigen allgemeinen Formel VI zur Reaktion bringt oder

h) eine racemische Verbindung der eingangs definierten allgemeinen Formel I in die optisch aktiven Antipoden aufspaltet oder

i) eine Verbindung der eingangs definierten allgemeinen Formel I in ein pharmazeutisch akzeptables Säureadditionssalz überführt.

Der Ausdruck « niederes Alkyl » bezeichnet gesättigte Kohlenwasserstoffreste mit höchstens 4 Kohlenstoffatomen, welche geradkettig oder verzweigt sein können, wie Methyl und dergleichen.

Der Ausdruck « niederes Alkoxy » — für sich allein genommen oder in der Kombination « niederes Alkoxycarbonyl » — bezeichnet eine niedere Alkyloxygruppe im Sinne der obigen Definition des Begriffs « niederes Alkyl ». Der Ausdruck « niederes Alkenyl » bezeichnet 3-5 Kohlenstoffatome enthaltende Kohlenwasserstoffreste, welche eine olefinische Doppelbindung enthalten und geradkettig oder verzweigt sein können, wie Allyl und dergleichen.

Der Ausdruck « niederes Alkanoyl » bezeichnet Acylreste von 2-4 Kohlenstoffatome enthaltenden Alkancarbonsäuren, welche geradkettig oder verzweigt sein können, wie Acetyl und dergleichen.

Die Ausdrücke « niederes Cycloalkylmethyl » und « niederes Cycloalkylcarbonyl » umfassen Radikale mit insgesamt höchstens 5 Kohlenstoffatomen, wie Cyclopropylmethyl, Cyclopropylcarbonyl und dergleichen.

Die N-Substitution der Verbindungen der allgemeinen Formel II oder Ia erfolgt nach an sich bekannten Methoden, beispielsweise mit einem dem einzuführenden Rest entsprechenden Halogenid oder Dialkylsulfat, vorzugsweise jedoch reduktiv z. B. mit einem dem einzuführenden Rest entsprechenden Aldehyd in Gegenwart von Ameisensäure oder durch Umsetzung mit einem dem einzuführenden Rest entsprechenden Aldehyd und anschliessende Behandlung mit einem geeigneten Reduktionsmittel, wie Natrium-cyanoborhydrid, Natriumborhydrid oder dergleichen.

So gelangt man beispielsweise ausgehend von einer Verbindung der Formel Ia, worin $R^{32}$ Methyl bedeutet, durch Umsetzung mit Allylbromid zu einer entsprechenden Verbindung der Formel I, worin $R^2$ Methyl und $R^3$ Allyl bedeuten. Mittels Formaldehyd und Ameisensäure erhält man beispielsweise aus einer Verbindung der Formel II, worin $R^{31}$ Wasserstoff bedeutet, oder aus einer Verbindung der Formel Ia, worin $R^{32}$ Methyl bedeutet, eine entsprechende Verbindung der Formel I, worin $R^2$ und $R^3$ je Methyl bedeuten, oder aus einer Verbindung der Formel II, worin $R^{31}$ Cyclopropylmethyl bedeutet, eine entsprechende Verbindung der Formel I, worin $R^2$ Methyl und $R^3$ Cyclopropylmethyl bedeuten.

Die Verfahrensparameter für die Durchführung der verschiedenen Ausführungsformen der N-Substitution von Verbindungen der Formel II und Ia sind jedem Fachmann geläufig. Natürlich ist zu beachten, dass nur solche Methoden verwendet werden, welche selektiv die gewünschte N-Substitution ergeben, wobei besonders zu beachten ist, dass bei Anwendung reduktiver Methoden nur solche in Frage kommen, welche die olefinische Doppelbindung im Cyclohexen-Teil des Moleküls nicht in Mitleidenschaft ziehen.

Die Reduktion der Verbindungen der allgemeinen Formel III oder IV erfolgt nach an sich bekannten Methoden. Selbstverständlich kommen nur solche Methoden in Betracht, welche selektiv zu den gewünschten entsprechenden Verbindungen der Formel I führen, wobei insbesondere zu beachten ist, dass die olefinische Doppelbindung im Cyclohexenteil des Moleküls nicht in Mitleidenschaft gezogen werden darf. Die Reduktion erfolgt zweckmässigerweise mittels sehr reaktiver komplexer Hydride, wie Lithiumaluminiumhydrid und Diisobutylaluminiumhydrid, in Gegenwart eines geeigneten inerten organischen Lösungsmittels, wie Aether, Tetrahydrofuran, Monoglym, Diglym oder dergleichen.

Die in Formel V durch das Symbol X wiedergegebene Abgangsgruppe kann ein Halogenatom sein, insbesondere Chlor, Brom oder Jod ; es kommen jedoch ohne weiteres auch äquivalente andere Abgangsgruppen in Betracht, z. B. Arylsulfonyloxyreste, wie Tosyloxy, Alkylsulfonyloxyreste, wie Mesyloxy, usw. Die Reaktionsparameter für die Umsetzung der Verbindungen der Formel V mit den Aminen der Formel VI sind jedem Fachmann geläufig. Man arbeitet zweckmässigerweise in Gegenwart eines geeigneten inerten organischen Lösungsmittels, z. B. in einem Alkohol, wie Methanol, Aethanol oder dergleichen, in Dimethylformamid, Toluol, Benzol, Xylol, Monoglym, Diglym oder dergleichen, und in Gegenwart eines säurebindenden Mittels, beispielsweise in Gegenwart einer anorganischen Base, wie Natriumcarbonat, Kaliumcarbonat oder dergleichen, eines tert. Amins, wie Triäthylamin, N-Aethyl-Diisopropylamin, Chinuclidin, Pyridin oder dergleichen ; als säurebindendes Mittel kann indessen auch ein Ueberschuss des als Reaktionskomponente eingesetzten Amins der allgemeinen Formel VI dienen.

Beim Erhitzen einer quartären Ammoniumbase der allgemeinen Formel VII oder eines geeigneten entsprechenden quartäres Salzes bzw. bei der Behandlung eines solchen Salzes mit einer Base erfolgt Spaltung des 5-gliedrigen heterocyclischen Rings und Einführung einer Doppelbindung im 6-gliedrigen alicyclischen Ring, und man erhält eine entsprechende Verbindung der Formel I, worin n die Zahl 1 und $R^2$ und $R^3$ je Methyl bedeuten.

Verwendet man als Ausgangsprodukt eine quartäre Ammoniumbase der allgemeinen Formel VII, so geht man zweckmässigerweise so vor, dass man diese Base in Abwesenheit eines Lösungsmittels auf eine Temperatur von etwa 100-200 °C, vorzugsweise etwa 160-180 °C, erhitzt und das gebildete entsprechende Amin der Formel I im Hochvacuum abdestilliert.

Von den den quartären Ammoniumbasen der allgemeinen Formel VII entsprechenden quartären Ammoniumsalzen eignen sich für den vorliegenden Verfahrensaspekt in erster Linie die Fluoride. Aus derartigen Fluoriden erhält man entsprechende Verbindungen der Formel I zweckmässigerweise dadurch, dass man sie in einem geeigneten Lösungsmittel, wie Acetonitril oder dergleichen, während etwa 1 Stunde erhitzt, vorzugsweise etwa auf Rückflusstemperatur des gewählten Lösungsmittels, oder dass man sie in einem geeigneten Lösungsmittel mit einer geeigneten Base behandelt, z. B. mit Natrium-t.-amylat in einem Gemisch von t.-Butanol und Toluol oder dergleichen, wobei man zweckmässigerweise bei etwa Raumtemperatur arbeitet.

Die Abspaltung der durch das Symbol $R^5$ bezeichneten niederen Alkylgruppe in den Verbindungen der Formel Ib erfolgt unter Anwendung von Methoden, welche jedem Fachmann für die Durchführung einer derartigen Aetherspaltung geläufig sind. Zweckmässigerweise verwendet man für diese Aetherspaltung saure Reagenzien, wie Bortribromid in einem geeigneten inerten organischen Lösungsmittel, z. B. in einem halogenierten Kohlenwasserstoff, wie Methylenchlorid, Chloroform usw., Bromwasserstoffsäure, Pyridinhydrochlorid oder dergleichen.

Als Schutzgruppen in den Verbindungen der Formel VIII (durch das Symbol $R^6$ bezeichnet) eignen sich selbstverständlich nur solche, welche durch Methoden abgespalten werden können, bei welchen diese Schutzgruppen selektiv entfernt werden, ohne dass andere im Molekül vorhandene Strukturelemente in Mitleidenschaft gezogen werden. Als derartige Schutzgruppen eignen sich beispielsweise leicht abspaltbare metallorganische Gruppen, insbesondere Trialkylsilylgruppen, wie Trimethylsilyl und dergleichen, leicht abspaltbare Acetal- und Ketalschutzgruppen, wie Hexahydropyran-2-yl und dergleichen usw. Die Entfernung der Schutzgruppe aus den Verbindungen der Formel VIII erfolgt nach an sich bekannten Methoden, wobei natürlich für die Wahl der zur Anwendung gelangenden Methode die Natur der zu entfernenden Schutzgruppe in Betracht gezogen werden muss und zu beachten ist, dass nur die Schutzgruppe selektiv entfernt, andere im Molekül vorhandene Strukturelemente jedoch nicht angegriffen werden sollen. So lassen beispielsweise die Trimethylsilylgruppe durch Behandeln mit verdünnter Salzsäure in Tetrahydrofuran oder dergleichen und die Hexahydropyran-2-ylgruppe unter milden sauren wässrigen Bedingungen (z. B. mittels 0,1 normaler Salzsäure) abspalten.

Die Verätherung einer Verbindung der allgemeinen Formel Ic erfolgt nach an sich bekannten, jedem Fachmann geläufigen Methoden, wobei natürlich zu beachten ist, dass die phenolische Hydroxylgruppe selektiv alkyliert, andere im Molekül vorhandene Strukturelemente jedoch nicht in Mitleidenschaft gezogen werden sollen. Die Verätherung erfolgt zweckmässigerweise mittels Diazoalkanen, wie Diazomethan in einem geeigneten inerten organischen Lösungsmittel, z. B. in einem Aether, wie Diäthyläther,

Tetrahydrofuran, Dioxan u.s.w., oder dergleichen, bei Temperaturen von etwa 0 °C bis etwa 50 °C, vorzugsweise etwa bei Raumtemperatur.

Methoden für die unter reduzierenden Bedingungen erfolgende Umsetzung einer Verbindung der Formel IX mit einem Amin der Formel VI sind jedem Fachmann geläufig, wobei natürlich zu beachten ist, dass nur solche Methoden in Frage kommen, bei welchen weder die im Cyclohexen-Teil der Verbindungen der Formel IX vorhandene olefinische Doppelbindung noch in den Aminen der Formel VI allfällig vorhandene reduzierbare Gruppen in Mitleidenschaft gezogen werden. Als Reduktionsmittel kommen Borhydride wie Natrium-cyanborhydrid, Natriumborhydrid oder dergleichen in Betracht. Zweckmässigerweise arbeitet man in Gegenwart eines geeigneten inerten organischen Lösungsmittels, z. B. in einem niederen Alkanol, wie Methanol, Aethanol, Isopropanol und dergleichen.

Die Aufspaltung racemischer Verbindungen der Formel I in die beiden optisch einheitlichen enantiomeren Komponenten erfolgt nach an sich bekannten Methoden, zweckmässigerweise durch Umsetzung mit einer optisch aktiven Säure, wie (+)-Di-O,O′-p-toluoyl-D-Weinsäure, und anschliessende Trennung der so erhaltenen diastereoisomeren Salze, beispielsweise durch fraktionierte Kristallisation, aus welchen die optisch einheitlichen Verbindungen der Formel I mittels Base freigesetzt werden können.

Die Ueberführung von Verbindungen der Formel I in pharmazeutisch akzeptable Säureadditionssalze erfolgt nach allgemein üblichen Methoden. Es kommen sowohl Salze mit anorganischen als auch Salze mit organischen Säuren in Betracht, beispielsweise Hydrochloride, Hydrobromide, Sulfate, Methansulfonate, p-Toluolsulfonate, Oxalate, Tartrate, Zitrate, Maleinate, Ascorbate, Acetate usw.

Zur Erläuterung der Herstellung der Zwischenprodukte der obigen allgemeinen Formeln II, III, IV, V, VII, VIII und IX wird zunächst auf die nachstehenden Reaktionsschemata verwiesen, in welchen n und X obige Bedeutung besitzen und $R^7$ niederes Alkoxy oder eine geschützte Hydroxygruppe bedeuten.

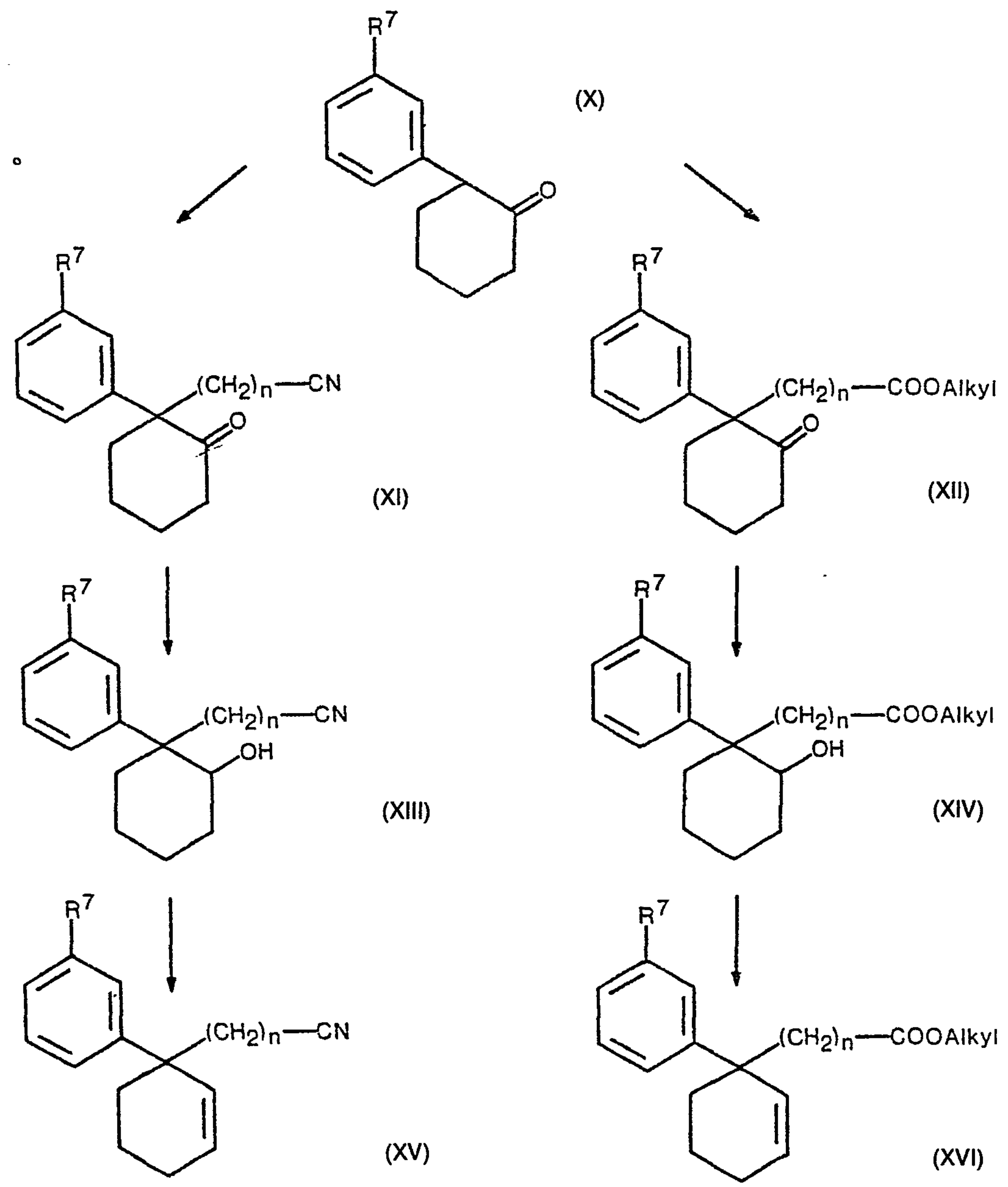

(X)

(XI) (XII)

(XIII) (XIV)

(XV) (XVI)

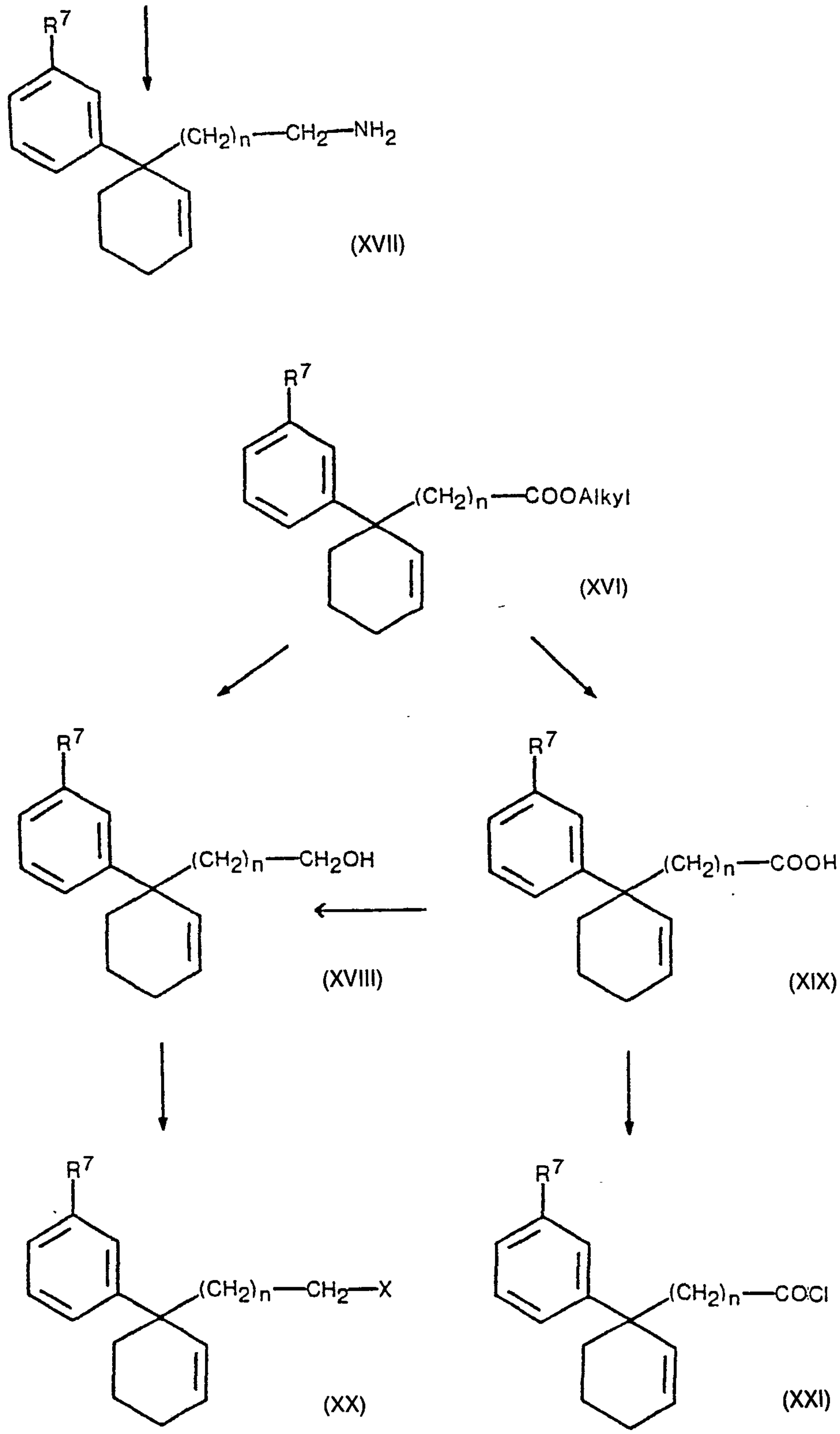

(XVII)

(XVI)

(XVIII)

(XIX)

(XX)

(XXI)

Die Verbindungen der allgemeinen Formel X, wie 2-(m-Methoxyphenyl)cyclohexanon, sind bekannt oder nach an sich bekannten und jedem Fachmann geläufigen Methoden zugänglich. Verbindungen der Formeln XI und XII erhält man aus den Verbindungen der Formel X durch Umsetzung mit Chloracetonitril, β-Chlorpropionitril, Acrylnitril oder dergleichen bzw. Bromessigsäureäthylester, β-Propionsäureäthylester, Acrylsäureäthylester oder dergleichen in Gegenwart einer starken Base. Verbindungen der Formeln XIII und XIV erhält man aus den Verbindungen der Formeln XI bzw. XII durch Reduktion, wobei die Reduktion der Verbindungen der Formel XI zweckmässigerweise mittels Natriumborhydrid oder

dergleichen und die Reduktion der Verbindungen der Formel XII zweckmässigerweise durch katalytische Hydrierung (beispielsweise über Platin) erfolgt. Verbindungen der Formeln XV und XVI erhält man aus den Verbindungen der Formel XIII bzw. XIV beispielsweise durch Behandlung mit Phosphoroxychlorid in Pyridin oder durch Umsetzung mit Methansulfochlorid und Behandlung des erhaltenen Produkts mit Kaliumacetat in Hexamethylphosphorsäuretriamid oder dergleichen. Verbindungen der Formeln XVII und XVIII erhält man aus den Verbindungen der Formel XV bzw. XVI oder XIX zweckmässigerweise durch Reduktion mit reaktiven komplexen Hydriden, wie Lithiumaluminiumhydrid, Diisobutylaluminiumhydrid oder dergleichen. Verbindungen der Formel XIX erhält man aus den Verbindungen der Formel XVI durch alkalische Hydrolyse, beispielsweise mittels Kaliumhydroxid in Aethanol/Wasser. Verbindungen der Formel XX erhält man aus den Verbindungen der Formel XVIII, indem man die Hydroxygruppe nach an sich bekannten Methoden durch eine Abgangsgruppe ersetzt, beispielsweise mittels Thionylchlorid, Tosylchlorid, Mesylchlorid oder dergleichen. Verbindungen der Formel XXI erhält man aus Verbindungen der Formel XIX nach an sich bekannten Methoden, beispielsweise mittels Oxalylchlorid, Thionylchlorid oder dergleichen.

Verbindungen der Formel XVII, worin $R^7$ niederes Alkoxy bedeutet, sind identisch mit Verbindungen der Formel II, worin $R^{31}$ Wasserstoff bedeutet. Die übrigen Verbindungen der Formel II sowie diejenigen der Formel Ia erhält man aus den Verbindungen der Formel XVII, worin $R^7$ niederes Alkoxy bedeutet, durch entsprechende N-Substitution, wobei zur Einführung von niederen Alkyl- oder niederen Cycloalkylmethylgruppen zweckmässigerweise so vorgegangen wird, dass man zunächst eine entsprechende Formyl-, Alkoxycarbonyl-, Alkanoyl- oder Cycloalkylcarbonylverbindung herstellt und diese anschliessend mit einem komplexen Hydrid, wie Lithiumaluminiumhydrid, Diisobutylaluminiumhydrid oder dergleichen, reduziert.

Verbindungen der Formeln II und Ia kann man auch dadurch herstellen, dass man Verbindungen der Formel XX, worin $R^7$ niederes Alkoxy bedeutet, mit Ammoniak oder einem entsprechenden Amin umsetzt oder dass man Verbindungen der Formel XXI, worin $R^7$ niederes Alkoxy bedeutet, mit Ammoniak oder einem entsprechenden primären Amin umsetzt und die erhaltene Verbindung mit einem komplexen Hydrid, wie Lithiumaluminiumhydrid, Diisobutylaluminiumhydrid oder dergleichen, reduziert.

Verbindungen der Formel III erhält man aus Verbindungen der Formel XVII durch entsprechende Mono- oder Disubstitution am Stickstoffatom nach an sich bekannten und jedem Fachmann geläufigen Methoden, worauf eine allfällig vorhandene Sauerstoff-Schutzgruppe abgespalten wird.

Verbindungen der Formel IV erhält man aus den Verbindungen der Formel XXI durch Umsetzung mit einem Amin der Formel VI nach an sich bekannten und jedem Fachmann geläufigen Methoden, worauf eine allfällig vorhandene Sauerstoff-Schutzgruppe abgespalten wird.

Verbindungen der Formel XX, worin $R^7$ niederes Alkoxy bedeutet, sind identisch mit den Verbindungen der Formel V.

Verbindungen der Formel VIII erhält man aus Verbindungen der Formel XVII, worin $R^7$ eine geschützte Hydroxygruppe bedeutet, durch entsprechende mono- oder Disubstitution am Stickstoff, welche nach an sich bekannten und jedem Fachmann geläufigen Methoden erfolgen kann. Verbindungen der Formel VIII kann man auch dadurch erhalten, dass man Verbindungen der Formel XX, worin $R^7$ eine geschützte Hydroxygruppe bedeutet, mit einem Amin der Formel VI umsetzt oder dass man eine Verbindung der Formel XXI, worin $R^7$ eine geschützte Hydroxygruppe bedeutet, mit einem Amin der Formel VI umsetzt und die erhaltene Verbindung anschliessend mit einem komplexen Hydrid, wie Lithiumaluminiumhydrid, Diisobutylaluminiumhydrid oder dergleichen, reduziert.

Verbindungen der Formel IX erhält man durch Oxidation von Verbindungen der Formel XVIII, zweckmässigerweise mittels Schwefeltrioxid-Pyridinkomplex in Dimethylsulfoxid, Oxalylchlorid und Dimethylsulfoxid oder anderen geeigneten Reagentien, und anschliessende Abspaltung einer allfällig vorhandenen Sauerstoff-Schutzgruppe.

Zur Herstellung von Verbindungen der Formel VII bzw. von geeigneten entsprechenden Salzen werden Verbindungen der Formel XI, worin n die Zahl 1 bedeutet, reduktiv zu Verbindungen der allgemeinen Formel

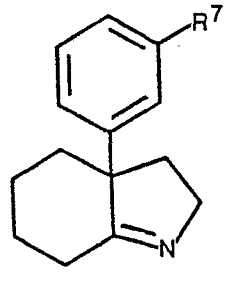

(XXII)

worin $R^7$ obige Bedeutung besitzt, cyclisiert, zweckmässigerweise durch katalytische Hydrierung (z. B. über Palladium). Hierauf wird die Kohlenstoffstickstoff-Doppelbindung reduziert, zweckmässigerweise mit Natriumborhydrid, Natrium-cyanborhydrid oder dergleichen, worauf man die erhaltene Verbindung nach an sich bekannten und jedem Fachmann geläufigen Methoden in das entsprechende quartäre

Dimethylammoniumhydroxid bzw. Dimethylammoniumsalz überführt, zweckmässigerweise durch Monomethylierung mittels Formaldehyd und Ameisensäure, anschliessende Quartärisierung mit Methyljodid und zuletzt Ersatz des Jodidions gegen das gewünschte Anion, was vorzugsweise mit einem geeigneten Anionenaustauscher erfolgt. Auf einer zweckmässigen Stufe der Synthese der quartären Dimethylammoniumbase bzw. des quartären Dimethylammoniumsalzes wird eine allfällig vorhandene Sauerstoff-Schutzgruppe abgespalten.

Verbindungen der Formel IV worin n die Zahl 1 und $R^2$ und $R^3$ je niederes Alkyl bedeuten, kann man nicht nur in der weiter oben beschriebenen Weise aus entsprechenden Verbindungen der Formeln XXI und VI erhalten, sondern auch dadurch, dass man eine Verbindung der allgemeinen Formel

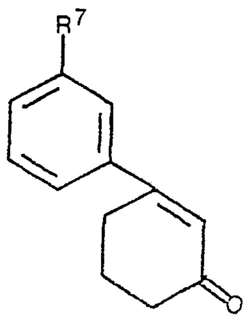

(XXIII)

worin $R^7$ obige Bedeutung besitzt, zu einer Verbindung der Formel

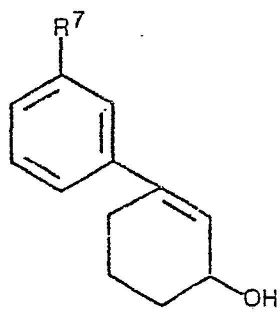

(XXIV)

worin $R^7$ obige Bedeutung besitzt, reduziert, zweckmässigerweise mittels Natriumborhydrid, Natriumcyanborhydrid oder dergleichen, und die so erhaltene Verbindung der Formel XXIV mit einem entsprechenden N,N-Dialkylacetamid-dialkylacetal, wie N,N-Dimethylacetamid-dimethylacetal, erhitzt, worauf dann eine allfällig vorhandene Sauerstoff-Schutzgruppe abgespalten wird.

Die Verbindungen der allgemeinen Formel XXIII sind bekannt oder nach an sich bekannten und jedem Fachmann geläufigen Methoden zugänglich.

Wie eingangs erwähnt, sind die Cyclohexen-Derivate der allgemeinen Formel I neue Verbindungen mit wertvollen pharmakodynamischen Eigenschaften. Wie im bekannten «Writhing-Test» gezeigt werden kann, sind sie analgetisch wirksam. In der nachfolgenden Tabelle werden die in diesem Test ermittelten ED 50-Werte für repräsentative Vertreter der durch die allgemeinen Formel I definierten Verbindungsklasse dargestellt; die Tabelle enthält ausserdem Angaben über die akute Toxizität der untersuchten Verbindungen (DL 50 in mg/Kg bei einmaliger oraler Verabreichung an Mäuse).

| Verbindung | ED 50 im "Writhing-Test" | DL 50 |
|---|---|---|
| rac.-1-(m-Methoxyphenyl)-N,N-dimethyl-2-cyclohexen-1-äthylamin-hydrochlorid | 33 mg/kg p.o. (nach 30 Min.) 35 mg/kg p.o. (nach 60 Min.) | 150-300 mg/kg |

(Fortsetzung)

| Verbindung | ED 50 im "Writhing-Test" | DL 50 |
|---|---|---|
| (+)-1-(m-Methoxyphenyl)-N,N-dimethyl-2-cyclohexen-1-äthylamin-hydrochlorid | 24 mg/kg p.o. (nach 60 Min.) | 250-500 mg/kg |
| (-)-1-(m-Methoxyphenyl)-N,N-dimethyl-2-cyclohexen-1-äthylamin-hydrochlorid | 19 mg/kg p.o. (nach 60 Min.) | 250-500 mg/kg |
| rac.-1-(m-Hydroxyphenyl)-N,N-dimethyl-2-cyclohexen-1-äthylamin-hydrochlorid | 41 mg/kg p.o. (nach 30 Min.) 26 mg/kg p.o. (nach 60 Min.) | 250-500 mg/kg |

Die Wirkungsstärke der obigen Verbindungen entspricht etwa derjenigen von Codein und Propoxyphen. Im Vergleich zu Codein und Propoxyphen zeichnen sie sich jedoch durch geringere unerwünschte Nebenwirkungen aus, insbesondere durch geringere bzw. fehlende Suchterzeugung.

Man kann Verbindungen der allgemeinen Formel I und pharmazeutisch akzeptable Säureadditionssalze davon bei der Bekämpfung bzw. Verhütung von Krankheiten verwenden, insbesondere bei der Bekämpfung von Schmerzen. Die Dosierung der Verbindungen der Formel I und ihrer pharmazeutisch akzeptablen Säureadditionssalze kann dabei innerhalb weiterer Grenzen variieren und ist natürlich in jedem einzelnen Fall den individuellen Gegebenheiten anzupassen. Im allgemeinen dürften bei oraler Verabreichung eine Einzeldosis von 100-300 mg und eine Tagesdosis von 400-1 200 mg angemessen sein.

Wie weiterhin eingangs erwähnt, sind Arzneimittel, enthaltend eine Verbindung der allgemeinen Formel I oder ein pharmazeutisch akzeptables Säureadditionssalz davon, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der allgemeinen Formel I oder pharmazeutisch akzeptable Säureadditionssalze davon und gegebenenfalls einen oder mehrere andere therapeutisch wertvolle Stoffe in eine galenische Darreichungsform bringt.

Die Verbindungen der allgemeinen Formel I und ihre pharmazeutisch akzeptablen Säureadditionssalze können als Heilmittel z. B. in Form pharmazeutischer Präparate Verwendung finden. Die pharmazeutischen Präparate können oral, z. B. in Form von Tabletten, Lacktabletten, Dragées, Hart- und Weichgelatinkapseln, Lösungen, Emulsionen oder Suspensionen, verabreicht werden. Die Verabreichung kann aber auch rektal, z. B. in Form von Supositorien, lokal oder perkutan, z. B. in Form von Salben, Crèmes, Gelées, Lösungen, oder parenteral, z. B. in Form von Injektionslösungen, erfolgen.

Zur Herstellung von Tabletten, Lacktabletten, Dragées und Hartgelatinekapseln können die Verbindungen der Formel I und ihre pharmazeutisch akzeptablen Säureadditionssalze mit pharmazeutisch inerten, anorganischen oder organischen Excipientien verarbeitet werden. Als solche Excipientien kann man z. B. für Tabletten, Dragées und Hartgelatinekapseln Lactose, Maisstärke oder Derivate davon, Talk, Stearinsäure oder deren Salze etc. verwenden.

Für Weichgelatinekapseln eignen sich als Excipientien z. B. vegetabile Oele, Wachse, Fette, halbfeste und flüssige Polyole etc. ; je nach Beschaffenheit des Wirkstoffes sind jedoch bei Weichgelatinekapseln überhaupt keine Excipientien erforderlich.

Zur Herstellung von Lösungen und Sirupen eignen sich als Excipientien z. B. Wasser, Polyole, Saccharose, Glukose und dergleichen.

Für Injektionslösungen eignen sich als Excipientien z. B. Wasser, Alkohole, Polyole, Glyzerin, vegetabile Oele etc.

Für Suppositorien, lokale oder perkutane Anwendungsformen eignen sich als Excipientien z. B. natürliche oder gehärtete Oele, Wachse, Fette, halbflüsige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

In den nachfolgenden Beispielen, welche die vorliegende Erfindung illustrieren, sind sämtliche Temperaturen in Celsiusgraden angegeben.

Biespiel 1

Zu 57 g Natriumhydrid (1,2 Mol, 50 %ige Dispersion in Oel) in 700 ml abs. Dimethylformamid tropft man unter Rühren und unter Stickstoffatmosphäre eine Lösung von 204 g (1,0 Mol) 2-(m-Methoxyphenyl)cyclohexanon in 150 ml Dimethylformamid innerhalb von ca. 30 Minuten, wobei die Temperatur auf ca. 40° ansteigt. Man rührt während einer weiteren Stunde, kühlt auf 10° und tropft dann unter Rühren eine Lösung von 76 ml Chloracetonitril (90,6 g/1,2 Mol) in 150 ml abs. Dimethylformamid zu, wobei man die Temperatur mittels Eiswasserkühlung bei 10-15° hält. Man rührt weitere 2 Stunden bei Raumtemperatur, gibt dann 50 ml Aethanol zu, giesst das Gemisch in ca. 2 l Eiswasser und extrahiert 2mal mit je 2 l Aether. Die ätherische Phase wird 3mal mit 250 ml Wasser gewaschen, über Magnesiumsulfat getrocknet und eingedampft. Das zurückbleibende braune Oel wird an 1 kg Kieselgel chromatographiert. Zunächst wird mit Aether/Petroläther 1 : 9 (ca. 10 l) eluiert, bis alle unpolaren Verunreinigungen herausgewaschen sind. Mit Aether/Petroläther 1 : 4 (ca. 15 l) wird das 1-(m-Methoxyphenyl)-2-oxo-cyclohexan-1-acetonitril eluiert. Die gemäss Dünnschichtchromatographie einheitlichen Fraktionen werden vereinigt, worauf man das Lösungsmittelgemisch abdestilliert und den kristallinen Rückstand aus Isopropyläther umkristallisiert. Erhalten werden farblose Kristalle von Smp. 62°.

243 g (1,0 Mol) 1-(m-Methoxyphenyl)-2-oxo-cyclohexan-1-acetonitril werden in 2,5 l Aethanol gelöst, worauf man unter Rühren portionenweise insgesamt 25 g (0,66 Mol) Natriumborhydrid zugibt. Die Temperatur steigt auf ca. 45° an. Nach 1 Stunde ist mittels Dünnschichtchromatographie kein 1-(m-Methoxyphenyl)-2-oxo-cyclohexan-1-acetonitril mehr nachweisbar. Nach Abdestillieren des Lösungsmittels versetzt man den Rückstand mit 500 ml Eiswasser und extrahiert 2mal mit je 1,5 l Aether. Man wäscht die vereinigten Extrakte nacheinander je 1mal mit 0,5 l Wasser, 0,5 l 1N Salzsäure und 0,5 l Wasser, trocknet sie über Magnesiumsulfat und destilliert das Lösungsmittel ab. Erhalten werden 240 g 1-(m-Methoxyphenyl)-2-hydroxy-cyclohexan-1-acetonitril in Form eines farblosen, zähen Oels, welches ohne Reinigung als Rohprodukt in 2 l Pyridin gelöst und mit 130 ml (1,4 Mol) Phosphoroxychlorid versetzt wird. Das Gemisch wird 3 Stunden am Rückfluss gekocht und anschliessend im Wasserstrahlvakuum eingedampft. Man versetzt den Rückstand unter Eiskühlung mit Wasser und extrahiert 2mal mit je 1,5 l Aether, worauf die ätherischen Phasen vereinigt, nacheinander je 1mal mit 0,5 l Wasser und 0,5 l 1N Salzsäure und anschliessend 2mal mit Wasser gewaschen, über Magnesiumsulat getrocknet und eingedampft werden. Das erhaltene braune Oel wird an der 10-fachen Menge Kieselgel gereinigt. Nach Elution der Verunreinigungen mit Aether/Petroläther 1 : 9 (ca. 10 l) wird mit Aether/Petroläther 1 : 3 (ca. 10 l) 1-(m-Methoxyphenyl)-2-cyclohexen-1-acetonitril eluiert. Die gemäss Dünnschichtchromatogramm einheitlichen Fraktionen werden vereinigt und das Lösungsmittel abdestilliert, wobei das Produkt als hellgelbes Oel zurückbleibt.

454 g (2,0 Mol) des obigen Oeles werden in 1,5 l abs. Tetrahydrofuran gelöst und bei Raumtemperatur unter Stickstoff-Atmosphäre zu einer Dispersion von 152 g (4,0 Mol) Lithiumaluminiumhydrid in 1,5 l abs. Tetrahydrofuran unter Rühren und unter gelegentlicher Eiskühlung so langsam zugetropft, dass die Temperatur 28° nicht übersteigt. Das Gemisch wird über Nacht bei Raumtemperatur weitergerührt und hierauf unter Eiskühlung und unter Stickstoff-Atmosphäre vorsichtig zuerst mit 50 ml Aethanol und dann mit 400 ml Tetrahydrofuran-Wasser (1 : 1) versetzt. Der erhaltene Niederschlag wird abgesaugt und gut mit Tetrahydrofuran gewaschen, worauf das Filtrat eingedampft wird. Das zurückbleibende zähe braune Oel wird mit 2 l 1N Salzsäure versetzt, worauf 3mal mit je 0,5 l Aether extrahiert wird. Die wässrige Phase wird durch Zugabe von konzentrierter Ammoniumhydroxidlösung unter Eiskühlung alkalisch gestellt und der ausgefallene Niederschlag in 2 l Aether aufgenommen. Die ätherische Lösung wird 1mal mit Wasser gewaschen, über Magnesiumsulfat getrocknet und eingedampft. Als Rückstand verbleibt 1-(m-Methoxyphenyl)-2-cyclohexen-1-äthylamin in Form eines hellbraunen Oels, welches ohne Reinigung als Rohprodukt weiterverarbeitet wird.

344 g (1,5 Mol) des obigen rohen Oels werden unter Eiskühlung mit einem Gemisch von 380 ml 90 %iger Ameisensäure (7,5 Mol) und 297 ml 35 %iger wässriger Formaldehydlösung (3,3 Mol) versetzt. Das Gemisch wird über Nacht bei 100° gerührt, dann abgekühlt und mit 310 ml 20 %iger Salzsäure (1,7 Mol) versetzt, worauf man zur Trockene eindampft. Man extrahiert den Rückstand 3mal mit einem Gemisch von je 500 ml Aethanol und 500 ml Benzol und dampft die vereinigten Extrakte zur Trockene ein. Das rohe Hydrochlorid des rac.-1-(m-Methoxyphenyl)-N,N-dimethyl-2-cyclohexen-1-äthylamins wird

aus Essigester unter Zusatz von wenig Aethanol umkristallisiert, und man erhält farblose Kristalle von Smp. 161-162°.

Beispiel 2

Durch Reduktion von 1-(m-Methoxyphenyl)-2-oxo-cyclohexan-propionitril mit Natriumborhydrid und anschliessende Dehydratisierung des erhaltenen 1-(m-Methoxyphenyl)-2-hydroxy-cyclohexan-1-propionitrils mit Phosphoroxychlorid in Pyridin in Analogie zu der in Beispiel 1 beschriebenen Herstellung von 1-(m-Methoxyphenyl)-2-cyclohexen-1-acetonitril erhält man 1-(m-Methoxyphenyl)-2-cyclohexen-1-propionitril in Form eines schwach gelblichen Oels.

Zu einer Suspension von 20 g Lithiumaluminiumhydrid in 300 ml abs. Tetrahydrofuran wird eine Lösung von 31,5 g 1-(m-Methoxyphenyl)-2-cyclohexen-1-propionitril in 100 ml abs. Tetrahydrofuran zugetropft. Das Reaktionsgemisch wird anschliessend während 4 Stunden am Rückfluss gekocht, abgekühlt und zunächst mit 20 ml Aethanol und dann mit einem Tetrahydrofuran-Wassergemisch (1 : 1) versetzt. Der ausgefallene Niederschlag wird abgesaugt und gut mit Methylenchlorid gewaschen, worauf das Filtrat eingedampft wird. Der ölige Rückstand wird mit einem Ueberschuss von 3N Salzsäure versetzt, worauf man mit Aether extrahiert, die saure wässrige Lösung durch Versetzen mit 3N Natronlauge alkalisch stellt und die Base in Methylenchlorid aufnimmt. Die organische Phase wird über Kaliumcarbonat getrocknet und eingedampft. Das als Rückstand verbleibende 1-(m-Methoxyphenyl)-2-cyclohexen-1-propylamin wird ohne Reinigung wie folgt weiterverarbeitet.

23,0 g des erhaltenen Produkts werden mit einem Gemisch von 23 ml 90 %iger Ameisensäure und 20 ml 37 %iger Formaldehydlösung versetzt und während 12 Stunden bei 100° gerührt. Nach dem Abkühlen wird mit 3N Natronlauge alkalisch gestellt und das rac.-1-(m-Methoxyphenyl)-N,N-dimethyl-2-cyclohexen-1-propylamin in Methylenchlorid aufgenommen. Das Oxalat dieser Verbindung bildet farblose Kristalle von Smp. 107-109°/nach Umlösen aus Essigester.

Beispiel 3

45 g 1-(m-Methoxyphenyl)-2-cyclohexen-1-äthylamin werden in 500 ml Ameisensäureäthylester gelöst und während 8 Stunden am Rückfluss gekocht. Nach Abdestillieren des überschüssigen Ameisensäureäthylesters wird das erhaltene Oel in Aether aufgenommen, worauf die organische Phase nacheinander mit 1N Salzsäure und Wasser gewaschen, über Magnesiumsulfat getrocknet und eingedampft wird. Das erhaltene N-/2-[1-(m-Methoxyphenyl)-2-cyclohexen-1-yl]äthyl/formamid, gelöst in 200 ml abs. Tetrahydrofuran, wird zu einer Dispersion von 24 g Lithiumaluminiumhydrid in 250 ml abs. Tetrahydrofuran zugetropft. Das Gemisch wird über Nacht am Rückfluss gekocht, dann auf Raumtemperatur abgekühlt und mit einem Gemisch von Tetrahydrofuran und Wasser (1 : 1) versetzt, worauf man den ausgefallenen Niederschlag absaugt und das Filtrat eindampft, wobei rac. 1-(m-Methoxyphenyl)-N-methyl-2-cyclohexen-1-äthylamin als Rückstand verbleibt. Das Hydrochlorid dieser Verbindung schmilzt bei 177-179° (farblose Kristalle, nach Umkristallisieren aus Essigester/-Methanol).

Beispiel 4

5,0 g 1-(m-Methoxyphenyl)-N-methyl-2-cyclohexen-1-äthylamin werden mit einem Gemisch aus 1,5 l 35 %iger wässriger Formaldehydlösung und 2,0 ml 90 %iger Ameisensäure versetzt und während 2 Stunden bei 100° gehalten. Nach Abdestillieren des Lösungsmittels versetzt man den Rückstand mit 1N Salzsäurelösung, nimmt den Neutralteil in Aether auf, stellt die wässrige Phase durch Zugabe von 3N Natronlauge alkalisch und nimmt das rac. 1-(m-Methoxyphenyl)-N,N-dimethyl-2-cyclohexen-1-äthylamin in Methylenchlorid auf. Nach dem Abdestillieren des Lösungsmittels wird das erhaltene Oel an der 10-fachen Menge Aluminiumoxid gereinigt. Die mit Toluol extrahierten Fraktionen werden zusammengefasst, und eingedampft, wobei reines rac. 1-(m-Methoxyphenyl)-N,N-dimethyl-2-cyclohexen-1-äthylamin als Rückstand verbleibt. Das Hydrochlorid dieser Verbindung schmilzt bei 161-162° (nach Umkristallisieren aus Essigester/Aethanol).

Beispiel 5

Man löst 2,5 g 1-(m-Methoxyphenyl)-N-methyl-2-cyclohexen-1-äthylamin in 50 ml Chloroform, versetzt diese Lösung mit einer Lösung von 1,65 g Chloral in 10 ml Chloroform, rührt das Gemisch bei Raumtemperatur über Nacht und gibt dann unter Kühlen 1N Salzsäure zu. Die organische Phase wird mit Wasser bis zur neutralen Reaktion gewaschen, über Magnesiumsulfat getrocknet und eingedampft. Das erhaltene ölige N-/2-[1-(m-Methoxyphenyl)-2-cyclohexen-1-yl]äthyl/-N-methylformamid wird ohne Reinigung wie folgt weiter verarbeitet :

Eine Lösung von 2,1 g dieses Oels in 50 ml abs. Tetrahydrofuran wird zu einer Suspension von 2,0 g Lithiumaluminiumhydrid in 20 ml abs. Tetrahydrofuran zugetropft, worauf das Gemisch über Nacht am Rückfluss gekocht, dann abgekühlt, mit Wasser versetzt und wie üblich aufgearbeitet wird. Man erhält rac. 1-(m-Methoxyphenyl)-N,N-dimethyl-2-cyclohexen-1-äthylamin. Das Hydrochlorid dieser Verbindung schmilzt bei 161-162° (farblose Kristalle nach Umkristallisieren aus Essigester/Aethanol).

Beispiel 6

4,9 g 1-(m-Methoxyphenyl)-N-methyl-2-cyclohexen-1-äthylamin werden in 50 ml Aceton gelöst. Hierauf gibt man 3,5 g Kaliumcarbonat zu, tropft unter Rühren langsam 1,8 ml Allylbromid zu und rührt das Reaktionsgemisch über Nacht. Anschliessend filtriert man den Niederschlag ab und dampft das Filtrat ein, wobei rac. N-Allyl-1-(m-methoxyphenyl)-N-methyl-2-cyclohexen-1-äthylamin als Rückstand verbleibt. Das Hydrochlorid dieser Verbindung schmilzt bei 114-116° (nach Umkristallisation aus Essigester/Isopropyläther).

Beispiel 7

3,45 g Cyclopropancarbonsäurechlorid werden in 50 ml Methylenchlorid vorgelegt, wonach man 5,0 g Kaliumcarbonat zugibt und dann unter Rühren eine Lösung von 7,0 g 1-(m-Methoxyphenyl)-2-cyclohexen-1-äthylamin in 10 ml Methylenchlorid zutropft. Das Gemisch wird über Nacht bei Raumtemperatur und anschliessend während 30 Minuten bei Rückflusstemperatur gerührt und dann abgekühlt, worauf man den Niederschlag absaugt, das Filtrat eindampft und das erhaltene Oel in Aether aufnimmt. Die ätherische Lösung wird mit 1N Salzsäure und anschliessend mit Wasser gewaschen, über Magnesiumsulfat getrocknet und eingedampft, wobei N-/2-[1-(m-Methoxyphenyl)-2-cyclohexen-1-yl]äthyl/cyclopropancarboxamid als Rückstand verbleibt. 6,5 g dieser Verbindung, gelöst in 30 ml abs. Tetrahydrofuran, werden zu einer Dispersion von 6 g Lithiumaluminiumhydrid in 100 ml abs. Tetrahydrofuran zugetropft, worauf während 3 Stunden am Rückfluss gekocht, abgekühlt und auf übliche Art aufgearbeitet wird. Man erhält N-(Cyclopropylmethyl)-1-(m-Methoxyphenyl)-2-cyclohexen-1-äthylamin ; Smp. des Hydrochlorids 141-142°.

Man versetzt 4,0 g N-(Cyclopropylmethyl)-1-(m-methoxyphenyl)-2-cyclohexen-1-äthylamin mit einem Gemisch von 1 ml 35 %iger wässriger Formaldehydlösung und 1,5 ml 90 %iger Ameisensäure, erhitzt während einer Stunde auf 100°, gibt dann 3N Salzsäure zu, destilliert das Lösungsmittel ab und kristallisiert den erhaltenen kristallinen Niederschlag aus Toluol um. Man erhält N-Cyclopropylmethyl-1-(m-methoxyphenyl)-N-methyl-2-cyclohexen-1-äthylamin-Hydrochlorid in Form farbloser Kristalle vom Smp. 140-141°.

Beispiel 8

10 g 3-(m-Methoxyphenyl)-2-cyclohexen-1-ol, erhalten durch Natriumborhydridreduktion von 3-(m-Methoxyphenyl)-2-cyclohexen-1-on, werden in 100 ml Xylol gelöst worauf man 12 g N,N-Dimethylacetamid-dimethylacetal zugibt und das Gemisch während 8 Stunden am Rückfluss kocht. Nach dem Abdestillieren des Lösungsmittels wird das zurückbleibende Oel an der 30-fachen Menge Kieselgel chromatographiert. Die durch Elution mit Methylenchlorid gewonnenen, gemäss Dünnschichtchromatogramm einheitlichen Fraktionen, werden zusammengefasst und eingedampft ; man erhält reines 1-(m-Methoxyphenyl)-N,N-dimethyl-3-cyclohexen-1-acetamid in Form eines schwach gelblichen Oels dar.

Zu einer Suspension von 1 g Lithiumaluminiumhydrid in 50 ml abs. Tetrahydrofuran gibt man eine Lösung von 1,0 g 1-(m-Methoxyphenyl)-N,N-dimethyl-3-cyclohexen-1-acetamid in 10 ml abs. Tetrahydrofuran und kocht das Reaktionsgemisch über Nacht am Rückfluss. Nach Zugabe von Wasser wird der ausgefallene Niederschlag abgesaugt und gut mit Tetrahydrofuran gewaschen, worauf man das Filtrat eindampft, das zurückbleibende Oel mit 1N Salzsäure versetzt, den Neutralteil in Aether aufnimmt, die saure Phase durch Zusatz von konz. Ammoniak alkalisch stellt und das freigesetzte rac.-1-(m-Methoxyphenyl)-N,N-dimethyl-2-cyclohexen-1-äthylamin in Methylenchlorid aufnimmt. Das Hydrochlorid dieser Verbindung schmilzt bei 161-162° (nach Umkristallisieren aus Essigester/Aethanol).

Beispiel 9

2,6 g rac. 1-(m-Methoxyphenyl)-N,N-dimethyl-2-cyclohexen-1-äthylamin, gelöst in 25 ml Chloroform, werden innerhalb von 2 Minuten bei − 50° unter Rühren zu einer Lösung von 5,8 ml Bortribromid in 175 ml Chloroform gegeben. Man rührt während einer halben Stunde bei − 50 °C weiter, lässt dann die Temperatur auf Raumtemperatur ansteigen und giesst hierauf das Reaktionsgemisch auf Eis. Durch Zugabe eines Ueberschusses an konz. Ammoniak wird das Reaktionsgemisch alkalisch gestellt, und anschliessend wird mit Chloroform ausgezogen. Die organische Phase wird mit Wasser gewaschen, über Magnesiumsulfat getrocknet und eingedampft, wobei rac. 1-(m-Hydroxyphenyl)-N,N-dimethyl-2-cyclohexen-1-äthylamin zurückbleibt. Das Hydrochlorid dieser Verbindung schmilzt bei 179-181° (nach Umlösen aus Methanol/Essigester). Die aus dem Hydrochlorid freigesetzte kristalline Base schmilzt bei 127-129° (nach Umlösen aus Methyläthylketon).

Beispiel 10

34,4 g rac. 1-(m-Methoxyphenyl)-N,N-dimethyl-2-cyclohexen-1-äthylamin werden in 250 ml abs. Aethanol gelöst und mit einer Lösung von 53,6 g (+)-Di-O,O'-p-toluoyl-D-weinsäure in 250 ml abs.

Aethanol versetzt. Man dampft das Gemisch ein, versetzt den Rückstand mit einem Gemisch von Aethanol und Benzol und dampft nochmals ein. Man versetzt den Rückstand mit 600 ml Essigester und erwärmt, bis eine klare Lösung entsteht, lässt diese über Nacht stehen und saugt den ausgefallenen Niederschlag ab. Das Filtrat wird auf 400 ml eingeengt und 6 Stunden stehengelassen, worauf der ausgefallene Niederschlag abgesaugt wird. Die vereinigten Kristallisate werden aus Aethanol/Aether umkristallisiert, und man erhält (+)-[1-(m-Methoxyphenyl)-N,N-dimethyl-2-cyclohexen-1-äthylamin]2,3-di-O-p-toluoyl-D-tartrat (1 : 1) von Smp. 138°, $[\alpha]_D$ = + 73,8° (c = 1, Methanol).

Man suspendiert das erhaltene Salz in Wasser und stellt mit einem Ueberschuss von 3N Natronlauge alkalisch. Die freigesetzte Base wird in Methylenchlorid aufgenommen, worauf die Methylenchloridlösung über Magnesiumsulfat getrocknet und eingedampft wird. Als Rückstand verbleibt (−)-1-(m-Methoxyphenyl)-N,N-dimethyl-2-cyclohexen-1-äthylamin in Form eines farblosen Oels, $[\alpha]_D$ = − 35,9° (c = 1, Methanol). Das Hydrochlorid dieser Verbindung erhält man in Form farbloser Kristalle vom Smp. 156-157°, $[\alpha]_D$ = − 32° (c = 1, Methanol).

Das nach Abtrennen des (+)-Di-toluoyl-D-tartrats erhaltene Filtrat wird eingedampft, worauf man den Rückstand mit einem Ueberschuss von 3N Natronlauge versetzt, die freigesetzte Base in Methylenchlorid aufnimmt und die Methylenchloridlösung eindampft. Der Rückstand wird in 200 ml abs. Aethanol gelöst, worauf man eine Lösung von 32,7 g (−)-Di-O,O'-p-toluoyl-L-weinsäure in 250 ml Aethanol zugibt, eindampft, den Rückstand mit einem Gemisch von Aethanol und Benzol versetzt und wiederum eindampft. Der erhaltene Rückstand wird in 150 ml Aethanol gelöst, und die Lösung wird über Nacht stehengelassen. Die ausgefallenen Kristalle werden abgesaugt und mit Essigester gewaschen. Man erhält (+)-[1-(m-Methoxyphenyl)-N,N-dimethyl-2-cyclohexen-1-äthylamin] 2,3-di-O-p-toluoyl-L-tartrat (1 : 1) vom Smp. 138°, $[\alpha]_D$ = 73,2° (c = 1, Methanol). Das wie oben beschrieben daraus freigesetzte (+)-1-(m-Methoxyphenyl)-N,N-dimethyl-2-cyclohexen-1-äthylamin stellt ein farbloses Oel dar, $[\alpha]_D$ = + 36,4° (c = 1, Methanol). Das Hydrochlorid dieser Verbindung zeigt einen Smp. von 156-157° (aus Aethanol/Aether), $[\alpha]_D$ = + 31,6° (c = 1, Methanol).

## Beispiel 11

200 g 1-(m-Methoxyphenyl)-2-oxocyclohexanessigsäureäthylester werden in 2 000 ml Aethanol gelöst und nach Versetzen mit 20 g Platinoxid bei 50° und 50 bar mit Wasserstoff reduziert. Nach Abtrennen des Katalysators wird das Lösungsmittel abdestilliert. Erhalten wird 2-Hydroxy-1-(m-methoxyphenyl) cyclohexanessigsäure-äthylester in Form eines hellgelben Oels.

In einem 3 lt-Rundkolben mit Thermometer, Rührer und Tropftrichter werden 195 g 2-Hydroxy-1-(m-methoxyphenyl) cyclohexanessigsäure-äthylester in 1 800 ml Methylenchlorid (über Molekularsieb, getrocknet) gelöst, mit 93,8 g Triäthylamin (0,93 mol) versetzt und das Reaktionsgemisch mit einem Eis-Methanolgemisch auf 0° abgekühlt. Unter Rühren wird dei dieser Temperatur innerhalb von ca. 30 Minuten eine Lösung von 78 g Methansulfochlorid in 400 ml Methylenchlorid so langsam zugetropft, dass die Temperatur 0° nicht übersteigt. Anschliessend wird während einer Stunde bei Raumtemperatur weitergerührt, worauf das Reaktionsgemisch auf 2 000 ml Eiswasser gegossen und die wässrige Phase mit 500 ml Methylenchlorid ausgeschüttelt wird. Die organische Phase wird zweimal mit je 250 ml Wasser gewaschen und über Magnesiumsulfat getrocknet. Das Lösungsmittel wird abdestilliert. Erhalten wird 1-(m-Methoxyphenyl)-2-[(methylsulfonyl)oxy]cyclohexanessigsäure-äthylester in Form eines schwach gelben Oels.

In einem 2 1/2 lt-Sulfierkolben mit Rührer, Thermometer und Rückflusskühler werden 100 g 1-(m-Methoxyphenyl)-2-[(methylsulfonyl)oxy]cyclohexanessigsäure-äthyl-ester in 1 000 ml Hexamethylphosphorsäuretriamid gelöst, die Lösung mit 290 g Kaliumacetat (geschmolzen und im Exsikkator aufbewahrt) versetzt und das Gemisch unter Stickstoffatmosphäre während 16 Stunden bei 100° gerührt.

Nach dem Abkühlen wird das Reaktionsgemisch auf 5 lt Eiswasser gegossen und zweimal mit je 2 lt Aether ausgeschüttelt. Die ätherische Lösung wird dreimal mit je 500 ml Wasser gewaschen und über Magnesiumsulfat getrocknet. Das Lösungsmittel wird abdestilliert. Erhalten wird ein gelbes Oel, das gemäss Gaschromatogramm aus 1-(m-Methoxyphenyl)-2-cyclohexen-1-essigsäure-äthylester und Octahydro-3a-(m-methoxyphenyl)benzofuran-2-on besteht und in 375 ml Aethanol gelöst wird, worauf man eine Lösung von 15,5 g Kaliumhydroxid in 155 ml Wasser zugibt und während 16 Stunden bei Raumtemperatur rührt. Nach Zugabe von 100 ml 3N Salzsäure wird das Lösungsmittel am Wasserstrahlvakuum zum grössten Teil abdestilliert. Der Rückstand wird mit 200 ml Eiswasser und 100 ml 3N Natronlauge versetzt und der Neutralteil in 2mal 500 ml Aether aufgenommen. Die ätherische Lösung wird zweimal mit je 50 ml Wasser gewaschen, worauf die vereinigten wässrigen Phasen durch Zugabe von 3N Salzsäure kongosauer gestellt werden. Die ausgefallene 1-(m-Methoxyphenyl)-2-cyclohexen-1-essigsäure wird in 2mal 1 000 ml Aether aufgenommen, worauf die ätherische Lösung zweimal mit je 100 ml Wasser gewaschen und über Magnesiumsulfat getrocknet wird. Das Lösungsmittel wird am Wasserstrahlvakuum abdestilliert, und man erhält ein gelbes Oel, das nach Versetzen mit 50 ml Isopropyläther kristallisiert. Die ausgefallenen Kristalle werden abgesaugt und bei 30° getrocknet. Die kristalline 1-(m-Methoxyphenyl)-2-cyclohexen-1-essigsäure zeigt einen Smp. von 59-61°.

38 g 1-(m-Methoxyphenyl)-2-cyclohexen-1-essigsäure werden in 500 ml Methylenchlorid (über Molekularsieb getrocknet) gelöst. Nach Versetzen mit 0,2 ml abs. Dimethylformamid werden bei 20° unter

Rühren und Eiskühlung innerhalb von ca. 30 Minuten 39,5 ml Oxalylchlorid zugetropft. Anschliessend rührt man eine Stunde bei Raumtemperatur. Nach dem Abdestillieren des Lösungsmittels werden zweimal je 250 ml Toluol zugegeben, worauf man das Lösungsmittel am Wasserstrahlvakuum abdestilliert. Das rohe 1-(m-Methoxyphenyl)-2-cyclohexen-1-essigsäurechlorid wird in einem 1 lt-Kolben mit Thermometer, Claisenaufsatz und Ammoniakkühler in 400 ml abs. Aether gelöst. Nach Abkühlen auf − 10° (Trockeneis Aceton) werden 100 ml Dimethylamin, das in einen Kolben mit Claisenaufsatz und Ammoniakkühler kondensiert wurde, in den die obige Aetherlösung von 1-(m-Methoxyphenyl)-2-cyclohexen-1-essigsäurechlorid enthaltenden Kolben destilliert. Das Reaktionsgemisch wird während einer Stunde bei − 10° und über Nacht bei Raumtemperatur weitergerührt, worauf man 200 ml Eiswasser zugibt und ausschüttelt. Die ätherische Lösung wird nacheinander mit 100 ml Wasser, 100 ml 1N Salzsäure, 100 ml Wasser, 100 ml 1N Natronlauge und zweimal je 100 ml Wasser gewaschen, über Magnesiumsulfat getrocknet und eingedampft. Man erhält rohes 1-(m-Methoxyphenyl)-N,N-dimethyl-2-cyclohexen-1-acetamid.

In einem 1 1/2 lt-Sulftierkolben mit Rückflusskühler, Thermometer, Rührer und Tropftrichter werden unter Stickstoffatmosphäre 10,0 g Lithiumaluminiumhydrid in 200 ml abs. Tetrahydrofuran vorgelegt und unter Rühren innerhalb ca. einer Stunde eine Lösung von 42 g 1-(m-Methoxyphenyl)-N,N-dimethyl-2-cyclohexen-1-acetamid in 450 ml abs. Tetrahydrofuran so zugetropft, dass die Temperatur 30° nicht übersteigt. Anschliessend wird das Reaktionsgemisch über Nacht bei einer Oelbadtemperatur 90° weitergerührt, auf Raumtemperatur abgekühlt und tropfenweise mit 50 ml Aethanol und anschliessend mit einem Gemisch von Tetrahydrofuran und Wasser (1 : 1) versetzt, bis eine weitere Zugabe dieses Gemisches zu keiner exothermen Reaktion mehr führt. Das Reaktionsgemisch wird dann mit 50 g Kaliumcarbonat versetzt, worauf der ausgefallene Niederschlag abgesaugt und mit Methylenchlorid nachgewaschen wird. Nach dem Abdestillieren des Lösungsmittels wird das erhaltene braune Oel mit 200 ml 1N Salzsäure versetzt und zweimal mit je 500 ml Aether extrahiert. Die ätherische Lösung wird einmal mit 100 ml Wasser gewaschen. Die wässrigen Phasen werden vereinigt und unter Eiskühlung durch Zugabe von konz. Ammoniumhydroxidlösung alkalisch gestellt. Die ausgefallene Base wird in 2mal je 500 ml Aether aufgenommen. Die organische Phase wird zweimal mit je 50 ml Kochsalzlösung gewaschen und über Magnesiumsulfat getrocknet, worauf das Lösungsmittel abdestilliert wird. Das erhaltene Oel wird an 400 g Aluminiumoxid (neutral) chromatographiert. Mit 2 500 ml Toluol eluiert man rac.-1-(m-Methoxyphenyl)-N,N-dimethyl-2-cyclohexen-1-äthylamin in Form eines farblosen Oels, das in 200 ml Aethanol gelöst und mit einem Ueberschuss an äthanolischer Salzsäure versetzt wird. Nach Abdestillieren des Lösungsmittels wird der Rückstand zweimal mit einem Gemisch von 100 ml Aethanol und 100 ml Toluol versetzt, worauf das Lösungsmittel am Wasserstrahlvakuum abdestilliert wird. Die erhaltenen Kristalle werden in einem Gemisch von Essigester und Aethanol (20 : 1) bei Siedehitze gelöst. Nach 6-stündigem Stehengelassen werden die ausgefallenen Kristalle abgesaugt. Man erhält rac.-1-(m-Methoxyphenyl)-N,N-dimethyl-2-cyclohexen-1-äthylamin-Hydrochlorid in Form farbloser Kristalle vom Smp. 161-162°.

## Beispiel 12

4,6 g Hexahydro-3a-(m-methoxyphenyl)-1-methylindolin werden in 100 ml Aceton gelöst und mit 10 ml Methyljodid versetzt. Das Gemisch wird während 10 Stunden am Rückfluss gekocht, worauf das Lösungsmittel abdestilliert und der Rückstand aus Aceton umkristallisiert wird. Das erhaltene Hexahydro-3a-(m-methoxyphenyl)-1,1-dimethylindoliniumjodid schmilzt bei 195-196°.

1 g Hexahydro-3a-(m-methoxyphenyl)-1,1-dimethyl-indoliniumjodid werden in 40 ml Wasser gelöst. Die Lösung wird auf eine Säule mit 50 g Amberlite IRA 400 (mit Natronlauge vorbehandelt ; bis zur neutraler Reaktion mit destilliertem Wasser gewaschen) aufgetragen, worauf mit 500 ml destilliertem Wasser eluiert wird. Nach Abdestillieren des Wassers wird das als Rückstand verbleibende Hexahydro-3a-(m-methoxyphenyl)-1,1-dimethyl-indoliniumhydroxid am Hochvakuum in einer Destillationsapparatur auf 170° erhitzt, wobei ein Oel überdestilliert. Dieses Oel enthält neben rac. 1-(m-Methoxyphenyl)-N,N-dimethyl-2-cyclohexen-1-äthylamin noch eine relativ geringe Menge Hexahydro-3a-(m-methoxyphenyl)-1-methylindolin.

## Beispiel 13

30 g Amberlite IRA 400 werden in 100 ml Wasser aufgeschlämmt und mit einer Lösung von 10 g Natriumfluorid in 100 ml Wasser versetzt. Nach 1-stündigem Rühren wird der Ionenaustauscher abgesaugt, mit Wasser gewaschen und auf eine Chromatographiesäule gebracht, worauf mit Wasser gewaschen wird, bis keine Fluoridionen mehr nachgewiesen werden können. Eine Lösung von 1 g Hexahydro-3a-(m-methoxyphenyl)-1,1-dimethyl-indoliniumjodid in 50 ml Wasser wird auf die Säule aufgetragen, worauf mit 500 ml Wasser eluiert wird.

Nach dem Abdestillieren des Lösungsmittels versetzt man das als Rückstand verbleibende Hexahydro-3a-(m-methoxyphenyl)-1,1-dimethyl-indoliniumfluorid 5mal mit einem Alkohol-Benzolgemisch (1 : 1, je 50 ml) und destilliert das Lösungsmittel jeweils wieder ab. Anschliessend wird der Rückstand im Hochvakuum bei 40° getrocknet, in 10 ml t-Butanol gelöst, mit 1,46 ml einer 1,935N Na-t-amylatlösung in

Toluol versetzt und während einer Stunde bei Raumtemperatur gerührt. Nach dem Abdestillieren des Lösungsmittels wird der Rückstand mit Wasser versetzt und das Produkt in Aether aufgenommen. Das erhaltene Oel enthält gemäss Gaschromatogramm 75 % 1-(m-Methoxyphenyl)-N,N-dimethyl-2-cyclohexen-1-äthylamin.

Beispiel 14

Zunächst verfährt man wie im ersten Absatz von Beispiel 13 beschrieben. Nach dem Abdestillieren des Lösungsmittels löst man das als Rückstand verbleibende Hexahydro-3a-(m-methoxyphenyl)-1,1-dimethyl-indoliniumfluorid in 10 ml Acetonitril und erhitzt während einer Stunde am Rückfluss. Nach Abdestillieren des Lösungsmittels wird der Rückstand mit Wasser versetzt, worauf die erhaltene Base in Aether aufgenommen und im Hochvakuum bei 150° destilliert wird. Das erhaltene Oel enthält gemäss dem Gaschromatogramm 86 % 1-(m-Methoxyphenyl)-N,N-dimethyl-2-cyclohexen-1-äthylamin.

Beispiel 15

Zu einer Suspension von 2,4 g Lithiumaluminiumhydrid in 50 ml abs. Tetrahydrofuran wird eine Lösung von 6,2 g 1-(m-Methoxyphenyl)-2-cyclohexen-1-essigsäure in 50 ml abs. Tetrahydrofuran zugetropft, worauf das Reaktionsgemisch über Nacht am Rückfluss gekocht wird. Nach dem Abkühlen auf Raumtemperatur versetzt man mit 20 ml Alkohol und anschliessend mit 50 ml einer wässrigen Tetrahydrofuranlösung (1 : 1), saugt den Niederschlag ab und dampft ein. Der Rückstand wird in Aether aufgenommen ; die organische Phase wird mit 1N Natronlauge und anschliessend mit Wasser ausgeschüttelt, über Magnesiumsulfat getrocknet und eingedampft. Das als Rückstand verbleibende 1-(m-Methoxyphenyl)-2-cyclohexen-1-äthanol stellt ein farbloses Oel dar.

Zu demselben Produkt gelangt man auch durch Reduktion von 1-(m-Methoxyphenyl)-2-cyclohexen-1-essigsäureäthylester mit Lithiumaluminiumhydrid.

1,0 g 1-(m-Methoxyphenyl)-2-cyclohexen-1-äthanol wird in 10 ml Methylenchlorid gelöst und mit 650 mg Triäthylamin versetzt. Unter Rühren und Kühlen auf 0° gibt man 540 mg Mesylchlorid zu und rührt anschliessend während einer Stunde bei Raumtemperatur. Das Reaktionsgemisch wird aus Eiswasser gegossen. Das 2-[1-(m-Methoxyphenyl)-2-cyclohexen-1-yl]-äthyl methansulfonat wird in Aether aufgenommen ; es stellt ein farbloses Oel dar.

1 g 2-[1-(m-Methoxyphenyl)-2-cyclohexen-1-yl]äthyl methansulfonat wird in 10 ml Isopropanol gelöst und mit 10 ml frisch destilliertem Dimethylamin versetzt. Das Gemisch wird mit einem Magnetrüher im Bombenrohr über Nacht bei Raumtemperatur gerührt. Nach Abdampfen des Lösungsmittels wird der Rückstand mit 1N Salzsäure versetzt ; die wässrige Phase wird mit Aether extrahiert und mit konz. Ammoniak versetzt, worauf das ausgefallene rac.-1-(m-Methoxyphenyl)-N,N-dimethyl-2-cyclohexen-1-äthylamin in Methylenchlorid aufgenommen wird. Das Hydrochlorid dieser Verbindung schmilzt bei 161-162°.

Beispiel 16

1,0 g 1-(m-Methoxyphenyl)-2-cyclohexen-1-äthanol wird in 1 ml Toluol gelöst. Nach Zugabe von 350 mg Pyridin und 540 mg Thionylchlorid wird während 3 Stunden bei Raumtemperatur gerührt, worauf das Reaktionsgemisch auf Eiswasser gegossen wird. Das 1-[1-(2-Chloräthyl)-2-cyclohexen-1-yl]-3-methoxybenzol wird in Aether aufgenommen ; es stellt ein farbloses Oel dar.

1 g 1-[1-(2-Chloräthyl)-2-cyclohexen-1-yl]-3-methoxybenzol wird in 10 ml Toluol gelöst und mit 10 ml Dimethylamin versetzt. Das Gemisch wird im Bombenrohr während 5 Stunden bei 150° gehalten. Nach dem Abdampfen des Lösungsmittels wird der Rückstand mit 3N Salzsäure versetzt. Die wässrige Phase wird mit Aether extrahiert und mit einem Ueberschuss an Ammoniak alkalisch gestellt, worauf das ausgefallene rac.-1-(m-Methoxyphenyl)-N,N-dimethyl-2-cyclohexen-1-äthylamin in Methylenchlorid aufgenommen wird. Das Hydrochlorid dieser Verbindung schmilzt bei 161-162°.

Beispiel 17

Eine Lösung von 1 ml Oxalylchlorid in 25 ml Methylenchlorid wird auf – 60° abgekühlt. Bei dieser Temperatur wird eine Lösung von 1,7 ml Dimethylsulfoxid in 5 ml Methylenchlorid unter Rühren zugetropft. Nach 5 Minuten wird bei – 60° eine Lösung von 2,32 g 1-(m-Methoxyphenyl)-2-cyclohexen-1-äthanol in 10 ml Methylenchlorid innerhalb von 5 Minuten zugetropft, worauf während 15 Minuten bei – 50° gerührt und nach Zugabe von 7 ml Triäthylamin weitere 5 Minuten bei – 50° gerührt wird. Nachdem das Reaktionsgemisch Zimmertemperatur erreicht hat, wird auf Eiswasser gegossen, und der 1-(m-Methoxyphenyl)-2-cyclohexen-1-acetaldehyd wird in Methylenchlorid aufgenommen. Die organische Phase wird mit Wasser gewaschen, über Magnesiumsulfat getrocknet und eingedampft. Die erhaltene Verbindung stellt ein farbloses Oel dar.

1,0 g 1-(m-Methoxyphenyl)-2-cyclohexen-1-acetaldehyd wird in 10 ml Methanol gelöst und nach Versetzen mit 10 ml Methylamin im Bombenrohr über Nacht bei Raumtemperatur mit einem Magnetrüher

gerührt. Nach dem Abdampfen des Lösungsmittels wird der Rückstand in Aethanol gelöst, worauf man mit 0,5 g Natriumborhydrid versetzt, während 2 Stunden bei Raumtemperatur rührt, das Lösungsmittel abdampft und den Rückstand mit 3N Salzsäure aufnimmt. Die wässrige Phase wird mit Aether ausgeschüttelt und durch Zugabe von konz. Ammoniak alkalisch gestellt, worauf das erhaltene rac.-1-(m-Methoxyphenyl)-N-methyl-2-cyclohexen-1-äthylamin in Methylenchlorid aufgenommen wird. Das Hydrochlorid dieser Verbindung schmilzt bei 177-179°.

Beispiel A

Hartgelatine-Kapsel :

a) Zusammensetzung :

| | |
|---|---|
| 1-(m-Methoxyphenyl)-N,N-dimethyl-2-cyclohexen-1-äthylamin-Hydrochlorid (Wirkstoff) | 100,0 mg |
| Milchzucker krist. | 102,0 mg |
| Maisstärke weiss | 45,0 mg |
| Talk | 10,4 mg |
| Magnesiumstearat | 2,6 mg |
| | 260,0 mg |

b) Herstellung :

Der Wirkstoff wird mit Maisstärke, Talk und Magnesiumstearat gemischt. Das Gemisch wird gesiebt, mit Milchzucker versetzt, gemischt und nochmals gesiebt. Die Pulvermischung wird in Kapseln geeigneter Grösse abgefüllt.

**Ansprüche** (für die Vertragsstaaten : BE, CH, DE, FR, GB, IT, LI, LU, NL, SE)

1. 1-Phenyl-2-cyclohexen-1-alkylamin-Derivate der allgemeinen Formel

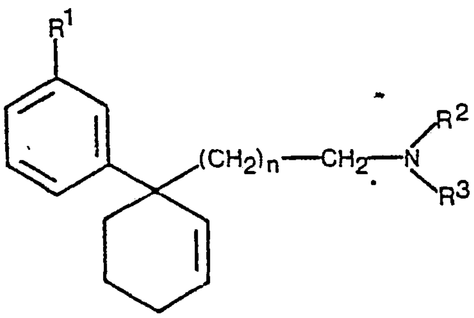

(I)

worin $R^1$ Hydroxy oder niederes Alkoxy, n die Zahl 1 oder 2, $R^2$ niederes Alkyl und $R^3$ Wasserstoff, niederes Alkyl, niederes Alkenyl oder niederes Cycloalkylmethyl bedeuten, und pharmazeutisch akzeptable Säureadditionssalze von Verbindungen der Formel I.

2. Verbindungen gemäss Anspruch 1, worin $R^1$ Hydroxy oder Methoxy, $R^2$ und $R^3$ je niederes Alkyl und n die Zahl 1 bedeuten.

3. Verbindungen gemäss Anspruch 1 oder 2, worin $R^2$ und $R^3$ je Methyl bedeuten.

4. 1-(m-Methoxyphenyl)-N,N-dimethyl-2-cyclohexen-1-äthylamin.

5. 1-(m-Hydroxyphenyl)-N,N-dimethyl-2-cyclohexen-1-äthylamin.

6. 1-(m-Methoxyphenyl)-N,N-dimethyl-2-cyclohexen-1-propylamin ; 1-(m-Methoxyphenyl)-N-methyl-2-cyclohexen-1-äthylamin ; N-Allyl-1-(m-methoxyphenyl)-N-methyl-2-cyclohexen-1-äthylamin ; N-Cyclopropylmethyl-1-(m-methoxyphenyl)-N-methyl-2-cyclohexen-1-äthylamin.

7. Verbindungen gemäss einem der Ansprüche 1 bis 6 als pharmazeutische Wirkstoffe.

8. Verbindungen gemäss einem der Ansprüche 1 bis 6 als analgetische Wirkstoffe.

9. 1-(m-Methoxyphenyl)-N,N-dimethyl-2-cyclohexen-1-äthylamin als pharmazeutischer Wirkstoff.

10. 1-(m-Methoxyphenyl)-N,N-dimethyl-2-cyclohexen-1-äthylamin als analgetischer Wirkstoff.

11. Verfahren zur Herstellung von Verbindungen gemäss einem der Ansprüche 1 bis 6, dadurch gekennzeichnet, dass man

a) eine Verbindung der allgemeinen Formel

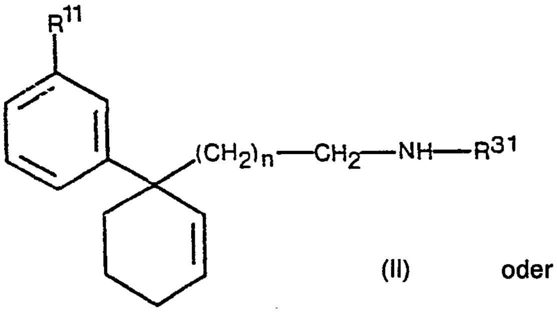

(II) oder

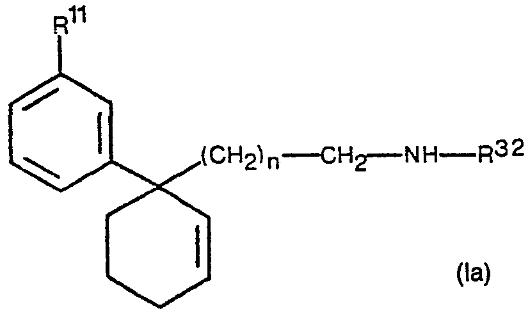

(Ia)

worin n die in Anspruch 1 angegebene Bedeutung besitzt und $R^{11}$ niederes Alkoxy, $R^{31}$ Wasserstoff, niederes Alkenyl oder niederes Cycloalkylmethyl und $R^{32}$ niederes Alkyl bedeuten, am Stickstoffatom entsprechend substituiert oder

b) eine Verbindung der allgemeinen Formel

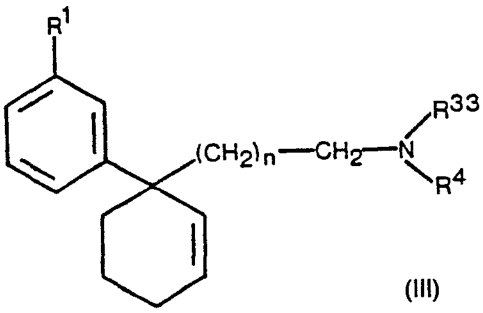

(III) oder

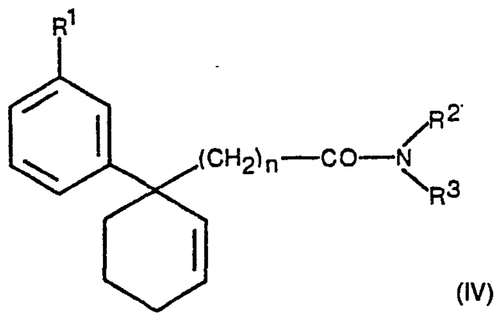

(IV)

worin $R^1$, $R^2$, $R^3$ und n die in Anspruch 1 angegebene Bedeutung besitzen und entweder $R^{33}$ Wasserstoff, niederes Alkyl, niederes Alkenyl oder niederes Cycloalkylmethyl und $R^4$ Formyl, niederes Alkanoyl oder niederes Alkoxycarbonyl oder $R^{33}$ niederes Alkyl und $R^4$ niederes Cycloalkylcarbonyl bedeuten, reduziert oder

c) eine Verbindung der allgemeinen Formel

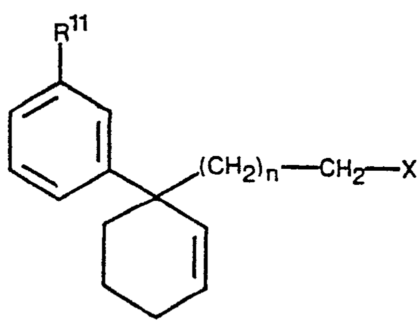

(V)

worin $R^{11}$ obige und n die in Anspruch 1 angegebene Bedeutung besitzen und X eine Abgangspruppe bedeutet, mit einem Amin der allgemeinen Formel

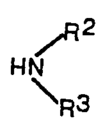

(IV)

worin $R^2$ und $R^3$ die in Anspruch 1 angegebene Bedeutung besitzen, umsetzt oder

d) eine quartäre Ammoniumbase der allgemeinen Formel

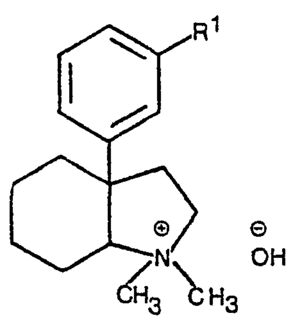

(VII)

worin $R^1$ die in Anspruch 1 angegebene Bedeutung besitzt, erhitzt oder ein geeignetes entsprechendes quartäres Ammoniumsalz erhitzt oder mit einer Base behandelt oder

e) aus einer Verbindung der allgemeinen Formel

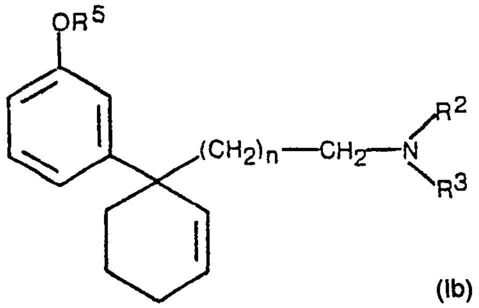

(Ib) oder

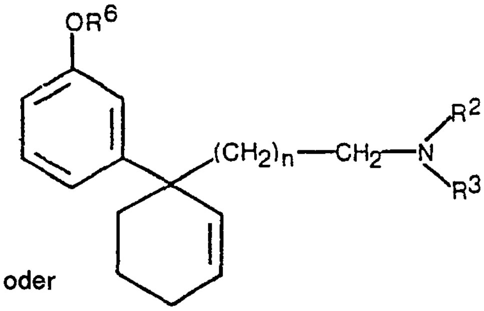

(VIII)

worin $R^2$, $R^3$ und n die in Anspruch 1 angegebene Bedeutung besitzen und $R^5$ niederes Alkyl und $R^6$ eine Schutzgruppe bedeuten, die Alkylgruppe $R^5$ bzw. die Schutzgruppe $R^6$ abspaltet oder

f) eine Verbindung der allgemeinen Formel

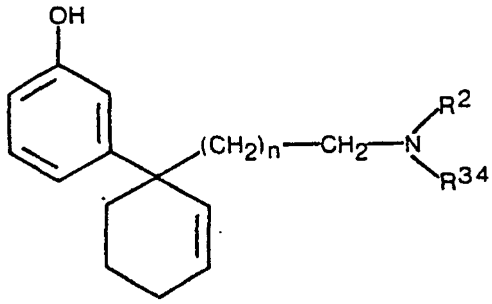

(Ic)

worin n und $R^2$ die in Anspruch 1 angegebene Bedeutung besitzen und $R^{34}$ niederes Alkyl, niederes Alkenyl oder niederes Cycloalkylmethyl bedeutet, veräther oder

g) eine Verbindung der allgemeinen Formel

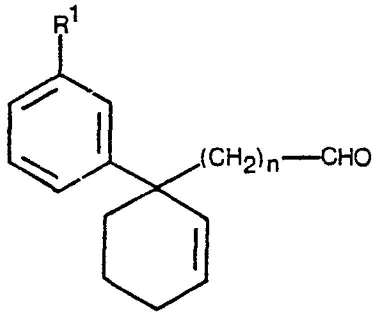

(IX)

worin $R^1$ und n die in Anspruch 1 angegebene Bedeutung besitzen, unter reduzierenden Bedingungen mit einem Amin der obigen allgemeinen Formel VI umsetzt oder

h) eine racemische Verbindung der in Anspruch 1 definierten allgemeinen Formel I in die optisch aktiven Antipoden aufspaltet oder

i) eine Verbindung der in Anspruch 1 definierten allgemeinen Formel I in ein pharmazeutisch akzeptables Säureadditionssalz überführt.

12. Verfahren nach Anspruch 11, dadurch gekennzeichnet, dass man 1-(m-Methoxyphenyl)-N,N-dimethyl-2-cyclohexen-1-äthylamin herstellt.

13. Arzneimittel, enthaltend eine Verbindung gemäss einem der Ansprüche 1 bis 6.

14. Analgetisches Mittel, enthaltend eine Verbindung gemäss einem der Ansprüche 1 bis 6.

15. Arzneimittel, enthaltend 1-(m-Methoxyphenyl)-N,N-dimethyl-2-cyclohexen-1-äthylamin.

16. Analgetisches Mittel, enthaltend 1-(m-Methoxyphenyl)-N,N-dimethyl-2-cyclohexen-1-äthylamin.

**Ansprüche** (für den Vertragsstaat AT)

1. Verfahren zur Herstellung von Cyclohexen-Derivaten der allgemeinen Formel

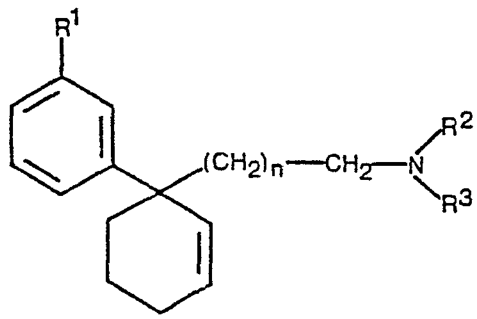

(I)

worin $R^1$ Hydroxy oder niederes Alkoxy, n die Zahl 1 oder 2, $R^2$ niederes Alkyl und $R^3$ Wasserstoff, niederes Alkyl, niederes Alkenyl oder niederes Cycloalkylmethyl bedeuten, und von pharmazeutisch akzeptablen Säureadditionssalzen von Verbindung der Formel I, dadurch gekennzeichnet, dass man

a) eine Verbindung der allgemeinen Formel

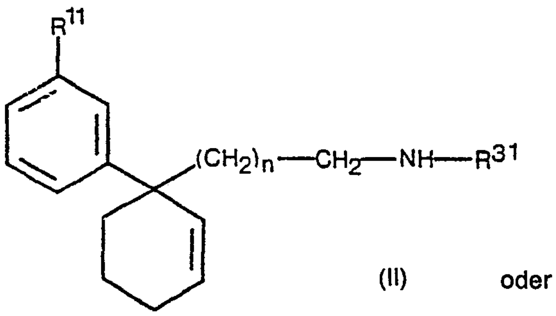

(II) oder

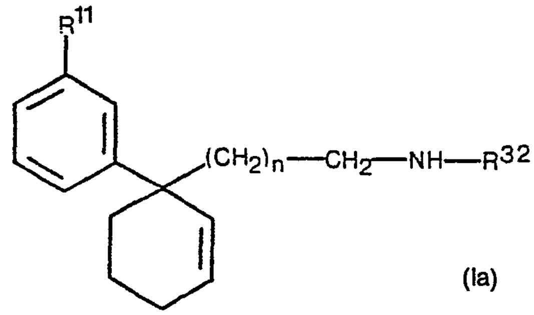

(Ia)

worin n obige Bedeutung besitzt und $R^{11}$ niederes Alkoxy, $R^{31}$ Wasserstoff, niederes Alkenyl oder niederes Cycloalkylmethyl und $R^{32}$ niederes Alkyl bedeuten, am Stickstoffatom entsprechend substituiert oder

b) eine Verbindung der allgemeinen Formel

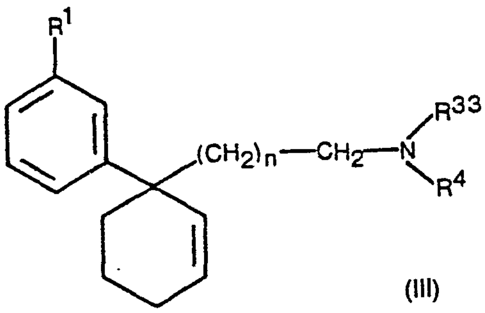

(III) oder

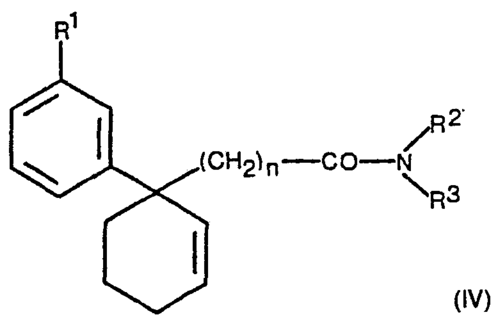

(IV)

worin $R^1$, $R^2$, $R^3$ und n obige Bedeutung besitzen und entweder $R^{33}$ Wasserstoff, niederes Alkyl, niederes Alkenyl oder niederes Cycloalkylmethyl und $R^4$ Formyl, niederes Alkanoyl oder niederes Alkoxycarbonyl oder $R^{33}$ niederes Alkyl und $R^4$ niederes Cycloalkylcarbonyl bedeuten, reduziert oder

c) eine Verbindung der allgemeinen Formel

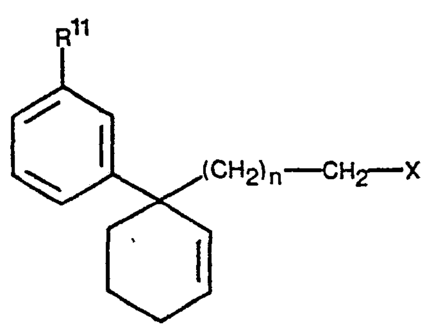

(V)

worin $R^{11}$ und n obige Bedeutung besitzen und X eine Abgangsgruppe bedeutet, mit einem Amin der allgemeinen Formel

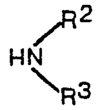

(VI)

worin $R^2$ und $R^3$ obige Bedeutung besitzen, umsetzt oder

d) eine quartäre Ammoniumbase der allgemeinen Formel

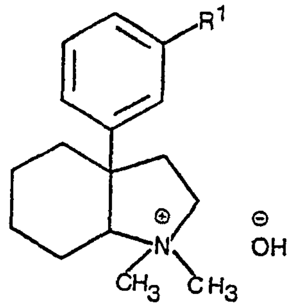

(VII)

worin $R^1$ obige Bedeutung besitzt, erhitzt oder ein geeignetes entsprechendes quartäres Ammoniumsalz erhitzt oder mit einer Base behandelt oder

e) aus einer Verbindung der allgemeinen Formel

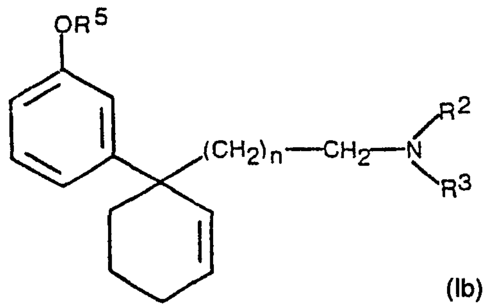

(Ib) oder

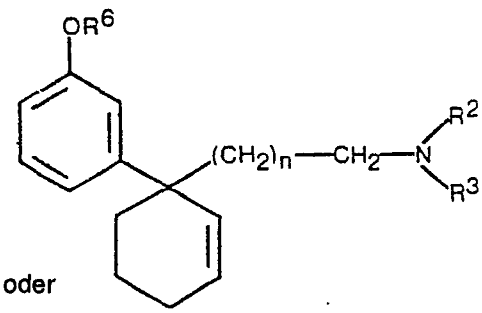

(VIII)

worin $R^2$, $R^3$ und n obige Bedeutung besitzen und $R^5$ niederes Alkyl und $R^6$ eine Schutzgruppe bedeuten, die Alkylgruppe $R^5$ bzw. die Schutzgruppe $R^6$ abspaltet oder.

f) eine Verbindung der allgemeinen Formel

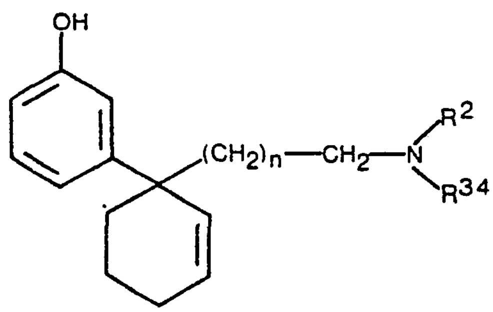

(Ic)

worin n und $R^2$ obige Bedeutung besitzen und $R^{34}$ niederes Alkyl, niederes Alkenyl oder niederes Cycloalkylmethyl bedeutet, veräthert oder

g) eine Verbindung der allgemeinen Formel

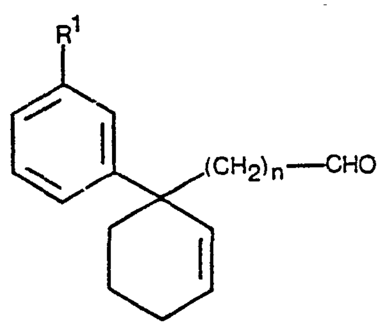

(IX)

worin $R^1$ und n obige Bedeutung besitzen, unter reduzierenden Bedingungen mit einem Amin der obigen allgemeinen Formel VI umsetzt oder

h) eine racemische Verbindung der allgemeinen Formel I in die optisch aktiven Antipoden aufspaltet oder

i) eine Verbindung der allgemeinen Formel I in ein pharmazeutisch akzeptables Säureadditionssalz überführt.

2. Verfahren nach Anspruch 1, dadurch gekennzeichnet, dass $R^1$ Hydroxy oder Methoxy, $R^2$ und $R^3$ je niederes Alkyl und n die Zahl 1 bedeuten.

3. Verfahren nach Anspruch 1 oder 2, dadurch gekennzeichnet, dass $R^2$ und $R^3$ je Methyl bedeuten.

4. Verfahren nach Anspruch 3, dadurch gekennzeichnet, dass man 1-(m-Methoxyphenyl)-N,N-dimethyl-2-cyclohexen-1-äthylamin herstellt.

5. Verfahren nach Anspruch 3, dadurch gekennzeichnet, dass man 1-(m-Hydroxyphenyl)-N,N-dimethyl-2-cyclohexen-1-äthylamin herstellt.

**Claims** (for the Contracting States : BE, CH, DE, FR, GB, IT, LI, LU, NL, SE)

1. 1-Phenyl-2-cyclohexene-1-alkylamine derivatives of the general formula

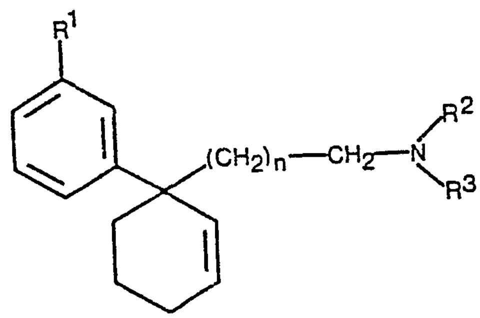

(I)

wherein $R^1$ signifies hydroxy or lower alkoxy, n signifies the number 1 or 2, $R^2$ signifies lower alkyl and $R^3$ signifies hydrogen, lower alkyl, lower alkenyl or lower cycloalkylmethyl, and pharmaceutically acceptable acid addition salts of compounds of formula I.

2. Compounds in accordance with claim 1, wherein $R^1$ signifies hydroxy or methoxy, $R^2$ and $R^3$ each signify lower alkyl and n signifies the number 1.

3. Compounds in accordance with claim 1 or 2, wherein $R^2$ and $R^3$ each signify methyl.

4. 1-(m-Methoxyphenyl)-N,N-dimethyl-2-cyclohexene-1-ethylamine.

5. 1-(m-Hydroxyphenyl)-N,N-dimethyl-2-cyclohexene-1-ethylamine.

6. 1-(m-Methoxyphenyl)-N,N-dimethyl-2-cyclohexene-1-propylamine ; 1-(m-methoxyphenyl)-N-methyl-2-cyclohexene-1-ethylamine ; N-allyl-1-(m-methoxyphenyl)-N-methyl-2-cyclohexene-1-ethylamine ; N-cyclopropylmethyl-1-(m-methoxyphenyl)-N-methyl-2-cyclohexene-1-ethylamine.

7. Compounds in accordance with any one of claims 1 to 6 as pharmaceutically active substances.

8. Compounds in accordance with any one of claims 1 to 6 as analgesically active substances.

9. 1-(m-Methoxyphenyl)-N,N-dimethyl-2-cyclohexene-1-ethylamine as a pharmaceutically active substance.

10. 1-(m-Methoxyphenyl)-N,N-dimethyl-2-cyclohexene-1-ethylamine as an analgesically active substance.

11. A process for the manufacture of compounds in accordance with any one of claims 1 to 6, characterized by

a) appropriately substituting a compound of the general formula

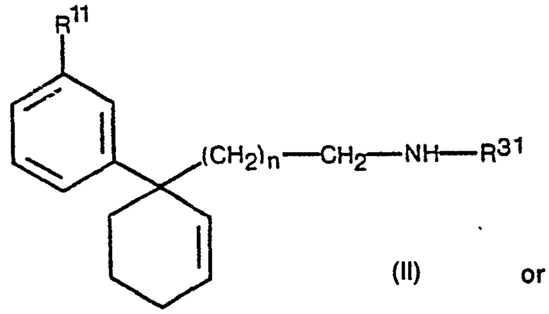

(II) or

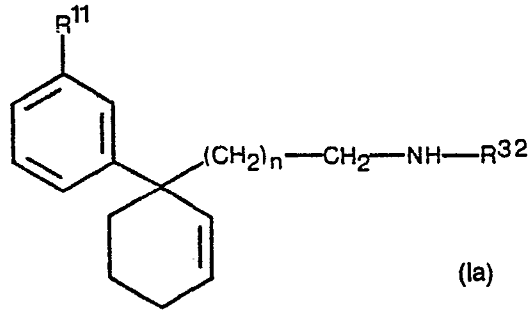

(Ia)

wherein n has the significance given in claim 1 and $R^{11}$ signifies lower alkoxy,

$R^{31}$ signifies hydrogen, lower alkenyl or lower cycloalkylmethyl and $R^{32}$ signifies lower alkyl, at the nitrogen atom, or

b) reducing a compound of the general formula

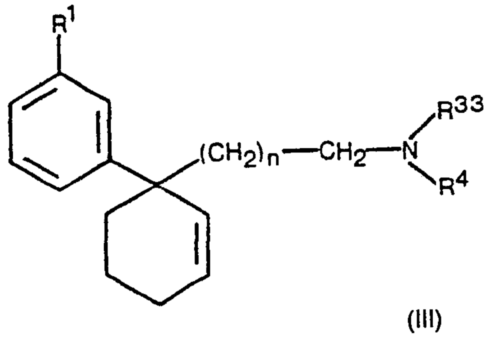

(III) or

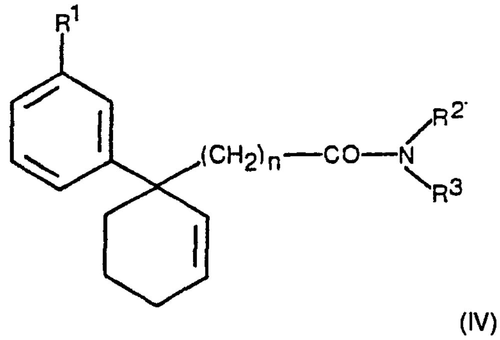

(IV)

wherein $R^1$, $R^2$, $R^3$ and n have the significance given in claim 1 and either $R^{33}$ signifies hydrogen, lower alkyl, lower alkenyl or lower cycloalkylmethyl and $R^4$ signifies formyl, lower alkanoyl or lower alkoxycarbonyl or $R^{33}$ signifies lower alkyl and $R^4$ signifies lower cycloalkylcarbonyl, or

c) reacting a compound of the general formula

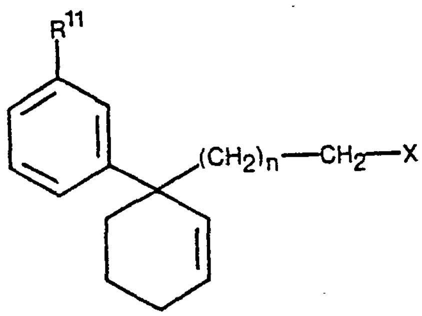

(V)

wherein $R^{11}$ has the significance given above and n has the significance given in claim 1 and X signifies a leaving group, with an amine of the general formula

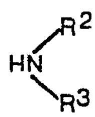

(VI)

wherein $R^2$ and $R^3$ have the significance given in claim 1, or

d) heating a quaternary ammonium base of the general formula

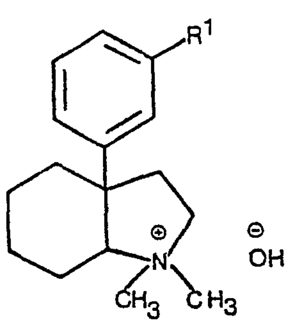

(VII)

wherein $R^1$ has the significance given in claim 1, or heating or treating with a base a suitable corresponding quaternary ammonium salt, or

e) cleaving off the alkyl group $R^5$ or the protecting group $R^6$ from a compound of the general formula

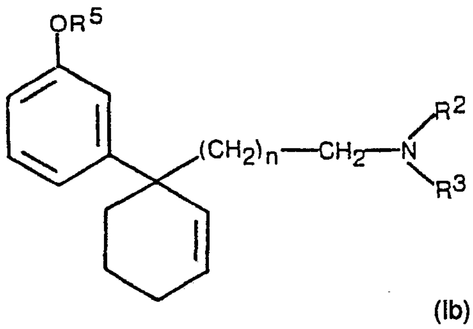

(Ib) or

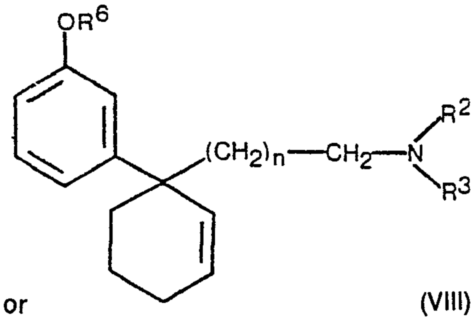

(VIII)

wherein $R^2$, $R^3$ and n have the significance given in claim 1 and $R^5$ signifies lower alkyl and $R_6$ signifies a protecting group, or

f) etherifying a compound of the general formula

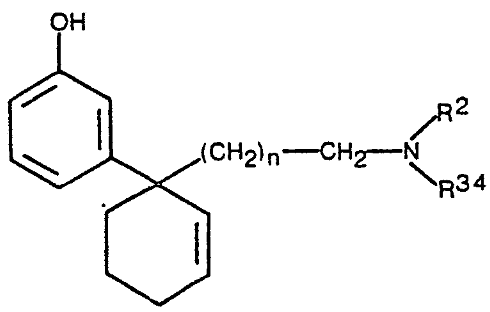

(Ic)

wherein n and $R^2$ have the significance given in claim 1 and $R^{34}$ signifies lower alkyl, lower alkenyl or lower cycloalkylmethyl, or

g) reacting a compound of the general formula

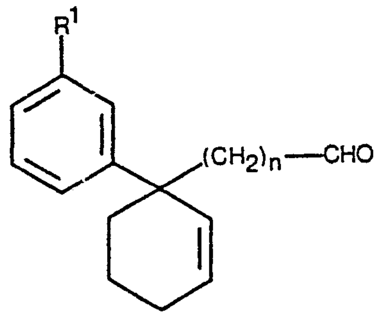

(IX)

wherein $R^1$ and n have the significance given in claim 1, under reducing conditions with an amine of general formula VI above, or

h) resolving a racemic compound of general formula I defined in claim 1 into the optically active antipodes, or

i) converting a compound of general formula I defined in claim 1 into a pharmaceutically acceptable acid addition salt.

12. A process according to claim 11, characterized in that 1-(m-methoxyphenyl)-N,N-dimethyl-2-cyclohexene-1-ethylamine is manufactured.

13. A medicament containing a compound in accordance with any one of claims 1 to 6.

14. An analgesic agent containing a compound in accordance with any one of claims 1 to 6.

15. A medicament containing 1-(m-methoxyphenyl)-N,N-dimethyl-2-cyclohexene-1-ethylamine.

16. An analgesic agent containing 1-(m-methoxyphenyl)-N,N-dimethyl-2-cyclohexene-1-ethylamine.

**Claims** (for the Contracting State AT)

1. A process for the manufacture of cyclohexene derivatives of the general formula

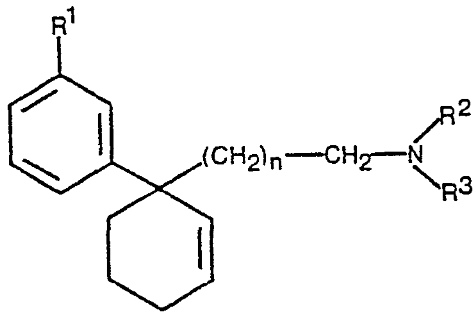

(I)

wherein $R^1$ signifies hydroxy or lower alkoxy, n signifies the number 1 or 2, $R^2$ signifies lower alkyl and $R^3$ signifies hydrogen, lower alkyl, lower alkenyl or lower cycloalkylmethyl, and of pharmaceutically acceptable acid addition salts of compounds of formula I, characterized by

a) appropriately substituting a compound of the general formula

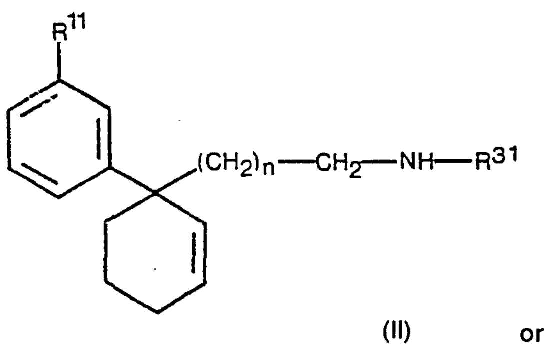

(II) or

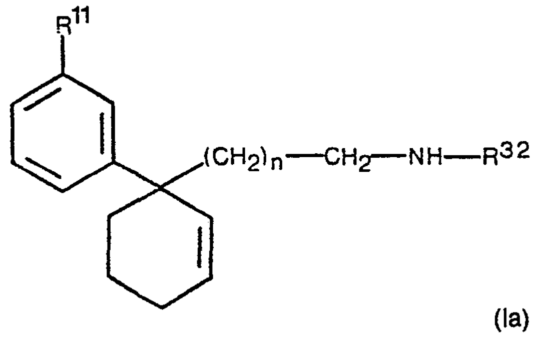

(Ia)

wherein n has the above significance and $R^{11}$ signifies lower alkoxy, $R^{31}$ signifies hydrogen, lower alkenyl or lower cycloalkylmethyl and $R^{32}$ signifies lower alkyl, at the nitrogen atom, or

b) reducing a compound of the general formula

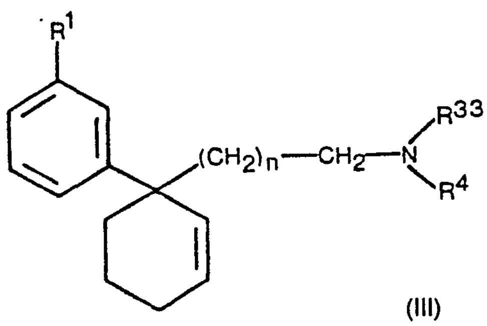

(III) or

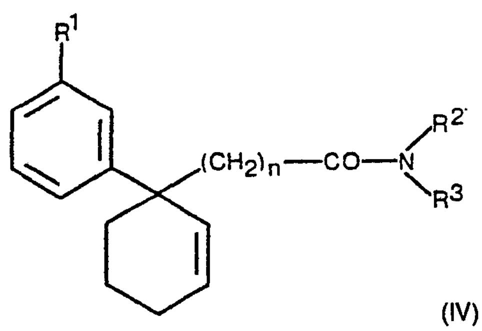

(IV)

wherein $R^1$, $R^2$, $R^3$ and n have the above significance and either $R^{33}$ signifies hydrogen, lower alkyl, lower alkenyl or lower cycloalkylmethyl and $R^4$ signifies formyl, lower alkanoyl or lower alkoxycarbonyl or $R^{33}$ signifies lower alkyl and $R^4$ signifies lower cycloalkylcarbonyl, or

c) reacting a compound of the general formula

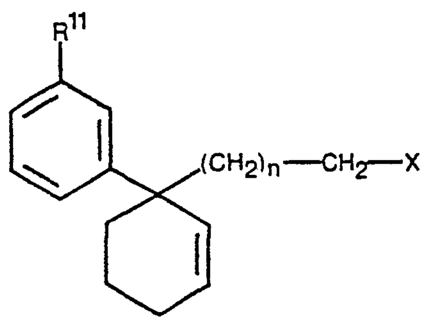

(V)

wherein $R^{11}$ and n have the above significance and X signifies a leaving group, with an amine of the

general formula

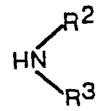

(VI)

wherein $R^2$ and $R^3$ have the above significance, or

d) heating a quaternary ammonium base of the general formula

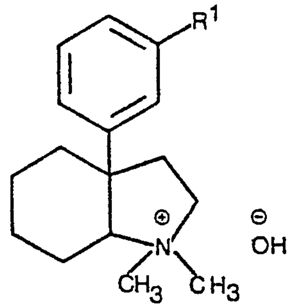

(VII)

wherein $R^1$ has the above significance, or heating or treating with a base a suitable corresponding quaternary ammonium salt, or

e) cleaving off the alkyl group $R^5$ or the protecting group $R^6$ from a compound of the general formula

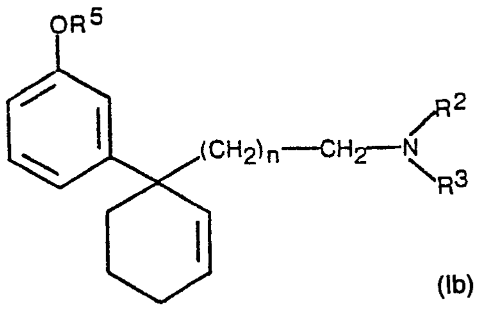

(Ib) or

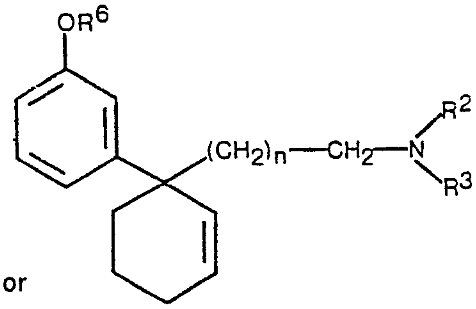

(VIII)

wherein $R^2$, $R^3$ and n have the above significance and $R^5$ signifies lower alkyl and $R^6$ signifies a protecting group, or

f) etherifying a compound of the general formula

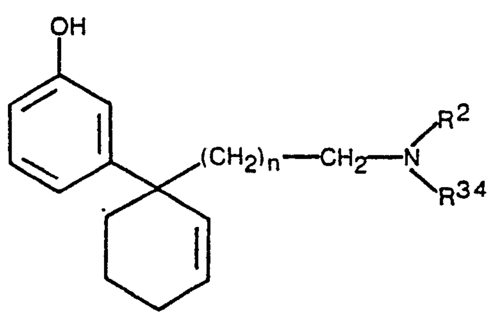

(Ic)

wherein n and $R^2$ have the above significance and $R^{34}$ signifies lower alkyl, lower alkenyl or lower cycloalkylmethyl, or

g) reacting a compound of the general formula

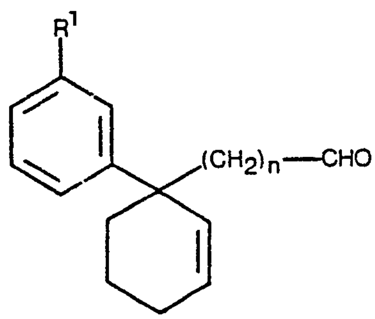

(IX)

wherein $R^1$ and n have the above significance, under reducing conditions with an amine of general formula VI above, or

h) resolving a racemic compound of general formula I into the optically active antipodes, or

i) converting a compound of general formula I into a pharmaceutically acceptable acid addition salt.

2. A process according to claim 1, characterized in that $R^1$ signifies hydroxy or methoxy, $R^2$ and $R^3$ each signify lower alkyl and n signifies the number 1.

3. A process according to claim 1 or 2, characterized in that $R^2$ and $R^3$ each signify methyl.

4. A process according to claim 3, characterized in that 1-(m-methoxyphenyl)-N,N-dimethyl-2-cyclohexene-1-ethylamine is manufactured.

5. A process according to claim 3, characterized in that 1-(m-hydroxyphenyl)-N,N-dimethyl-2-cyclohexene-1-ethylamine is manufactured.

**Revendications** (pour les Etats contractants : BE, CH, DE, FR, GB, IT, LI, LU, NL, SE)

1. Dérivés de 1-phényl-2-cyclohexène-1-alkylamines de formule générale

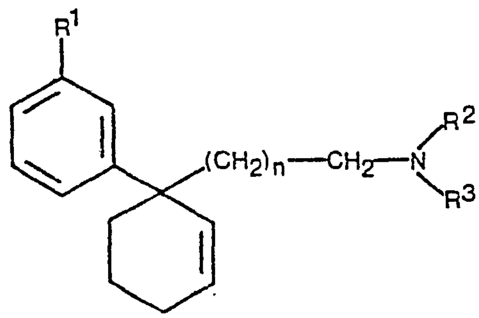

(I)

dans laquelle $R^1$ représente un groupe hydroxy ou alcoxy inférieur, n est égal à 1 ou 2, $R^2$ représente un groupe alkyle inférieur et $R^3$ l'hydrogène, un groupe alkyle inférieur, alcényle inférieur ou cycloalkylméthyle inférieur, et les sels des composés de formule I formés par addition avec des acides acceptables pour l'usage pharmaceutique.

2. Composés selon la revendication 1, dans lesquels $R^1$ représente un groupe hydroxy ou méthoxy, $R^2$ et $R^3$ représentent chacun un groupe alkyle inférieur et n est égal à 1.

3. Composés selon la revendication 1 ou 2, dans lesquels $R^2$ et $R^3$ représentent tous deux des groupes méthyle.

4. La 1-(m-méthoxyphényl)-N,N-diméthyl-2-cyclohexène-1-éthylamine.

5. La 1-(m-hydroxyphényl)-N,N-diméthyl-2-cyclohexène-1-éthylamine.

6. La 1-(m-méthoxyphényl)-N,N-diméthyl-2-cyclohexène-1-propylamine ; la 1-(m-méthoxyphényl)-N-méthyl-2-cyclohexène-1-éthylamine ; la N-allyl-1-(m-méthoxyphényl)-N-méthyl-2-cyclohexène-1-éthylamine ; la N-cyclopropylméthyl-1-(m-méthoxyphényl)-N-méthyl-2-cyclohexène-1-éthylamine.

7. Composés selon l'une des revendications 1 à 6, en tant que substances actives pharmaceutiques.

8. Composés selon l'une des revendications 1 à 6, en tant que substances actives analgésiques.

9. La 1-(m-méthoxyphényl)-N,N-diméthyl-2-cyclohexène-1-éthylamine en tant que substance active pharmaceutique.

10. La 1-(m-méthoxyphényl)-N,N-diméthyl-2-cyclohexène-1-éthylamine en tant que substance active analgésique.

11. Procédé de préparation des composés selon l'une des revendications 1 à 6, caractérisé en ce que :

a) on procède à la substitution correspondante sur l'atome d'azote d'un composé de formule générale

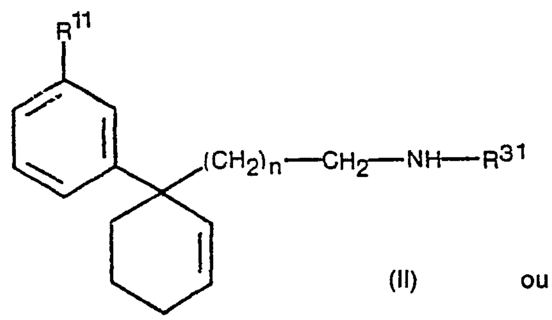

(II) ou

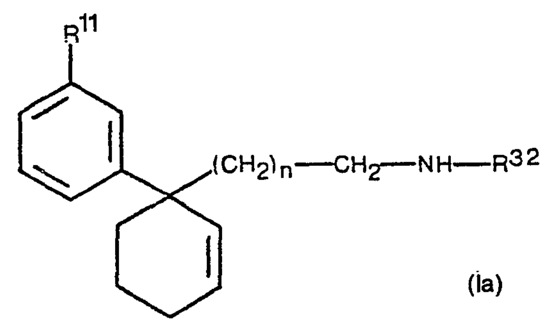

(Ia)

dans laquelle n a la signification indiquée dans la revendication 1 et $R^{11}$ représente un groupe alcoxy inférieur, $R^{31}$ représente l'hydrogène, un groupe alcényle inférieur ou cycloalkylméthyle inférieur et $R^{32}$ représente un groupe alkyle inférieur, ou bien

b) on réduit un compoé de formule générale

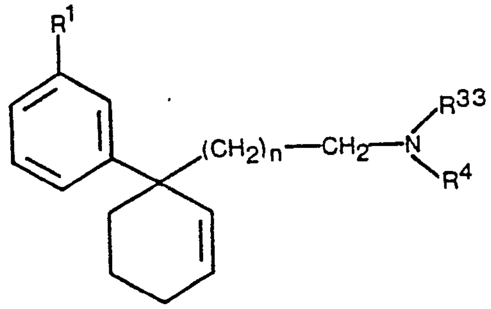

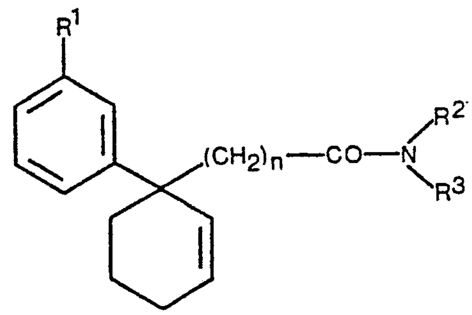

(III) ou (IV)

dans laquelle $R^1$, $R^2$, $R^3$ et n ont les significations indiquées dans la revendication 1 et ou bien $R^3$ représente l'hydrogène, un groupe alkyle inférieur, alcényle inférieur ou cycloalkylméthyle inférieur et $R^4$ représente un groupe formyle, alcanoyle inférieur ou alcoxycarbonyle inférieur, ou bien $R^{33}$ représente un groupe alkyle inférieur et $R^4$ un groupe cycloalkylcarbonyle inférieur, ou bien

c) on fait réagir un composé de formule générale

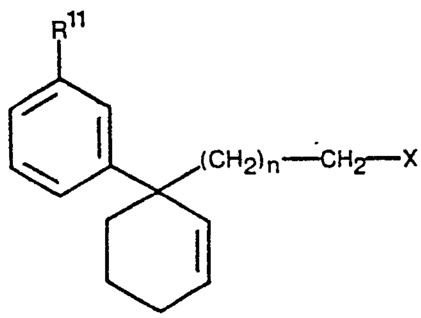

(V)

dans laquelle $R^{11}$ a la signification indiquée ci-dessus et n la signification indiquée dans la revendication 1, X représentant un groupe éliminable, avec une amine de formule générale

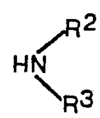

(VI)

dans laquelle $R^2$ et $R^3$ ont les significations indiquées dans la revendication 1, ou bien

d) on chauffe une base d'ammonium quaternaire de formule générale

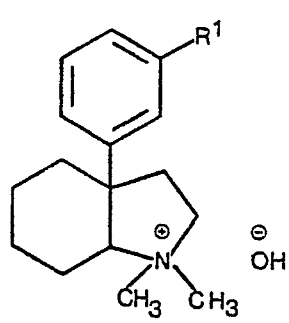

(VII)

dans laquelle $R^1$ a la signification indiquée dans la revendication 1, ou bien on chauffe un sel d'ammonium quaternaire correspondant approprié ou on le traite par une base, ou bien

e) dans un composé de formule générale

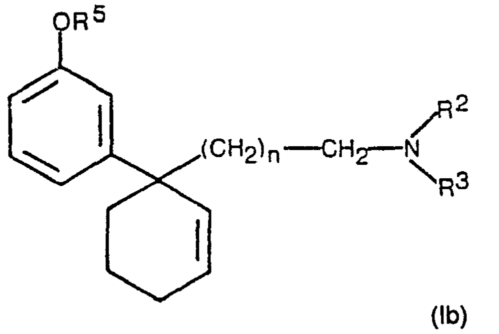

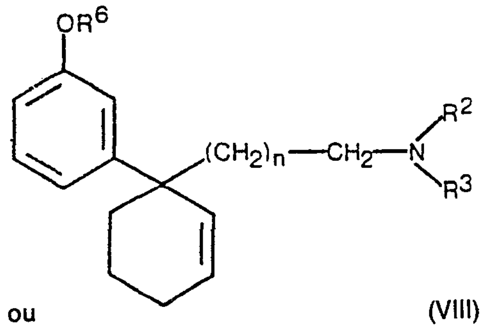

(Ib) ou (VIII)

dans laquelle $R^2$, $R^3$ et n ont les significations indiquées dans la revendication 1 et $R^5$ représente un groupe alkyle inférieur et $R^6$ un groupe protecteur, on élimine respectivement le groupe alkyle $R^5$ ou le groupe protecteur $R^6$, ou bien

f) on éthérifie un composé de formule générale

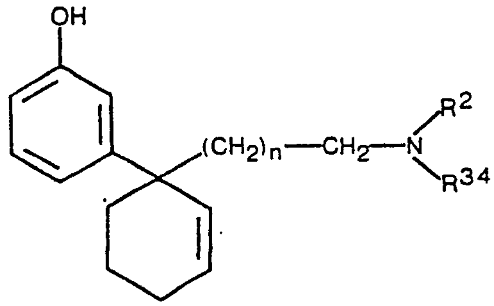

(Ic)

dans laquelle n et $R^2$ ont les significations indiquées dans la revendication 1 et $R^{34}$ représente un groupe alkyle inférieur, alcényle inférieur ou cycloalkylméthyle inférieur ou bien

g) on fait réagir un composé de formule générale

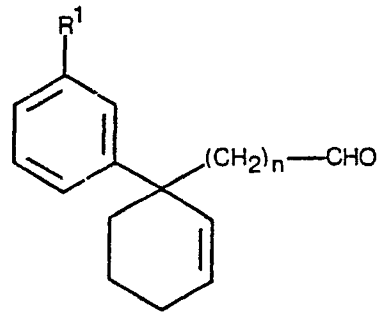

(IX)

dans laquelle $R^1$ et n ont les significations indiquées dans la revendication 1, dans des conditions réductrices, avec une amine de formule générale VI ci-dessus, ou bien

h) on résout un composé racémique de formule générale I définie dans la revendication 1 en les antipodes optiques, ou bien

i) on convertit un composé de formule générale I définie dans la revendication 1 en un sel formé par addition avec un acide acceptable pour l'usage pharmaceutique.

12. Procédé selon la revendication 11, caractérisé en ce que l'on prépare la 1-(m-méthoxyphényl)-N,N-diméthyl-2-cyclohexène-1-éthylamine.

13. Médicament contenant un composé selon l'une des revendications 1 à 6.

14. Agent analgésique contenant un composé selon l'une des revendications 1 à 6.

15. Médicament contenant de la 1-(m-méthoxyphényl)-N,N-diméthyl-2-cyclohexène-1-éthylamine.

16. Agent analgésique contenant de la 1-(m-méthoxyphényl)-N,N-diméthyl-2-cyclo-hexène-1-éthylamine.

**Revendications** (pour l'Etat contractant AT)

1. Procédé de préparation de dérivés du cyclohexène de formule générale

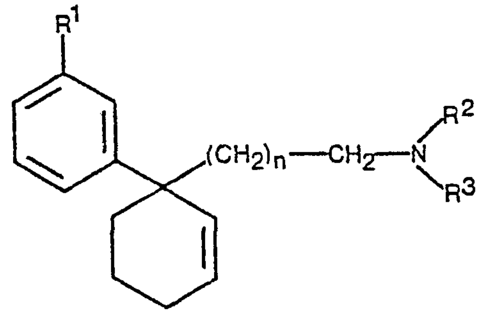

(I)

dans laquelle $R^1$ représente un groupe hydroxy ou alcoxy inférieur, n est égal à 1 ou 2, $R^2$ représente un groupe alkyle inférieur et $R^3$ l'hydrogène, un groupe alkyle inférieur, alcényle inférieur ou cycloalkylméthyle inférieur, et des sels d'un composé de formule I, formés par addition avec des acides acceptables pour l'usage pharmaceutique, caractérisé en ce que :

a) on soumet à la substitution correspondante sur l'atome d'azote un composé de formule générale

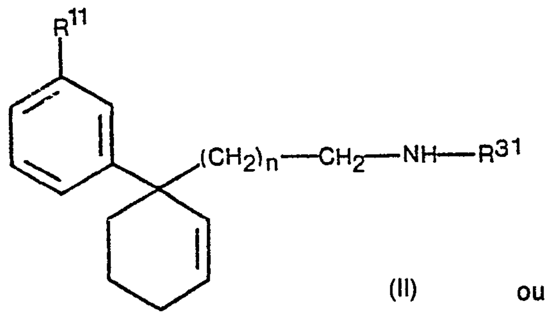

(II) ou

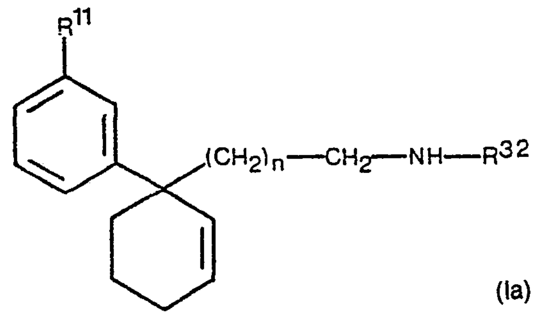

(Ia)

dans laquelle n a la signification indiquée ci-dessus et $R^{11}$ représente un groupe alcoxy inférieur, $R^{31}$ représente l'hydrogène, un groupe alcényle inférieur ou cycloalkylméthyle inférieur et $R^{32}$ représente un groupe alkyle inférieur, ou bien

b) on réduit un composé de formule générale

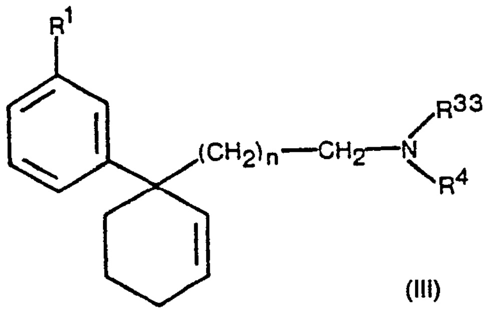

(III) ou

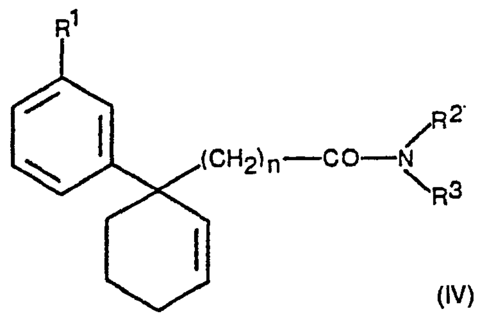

(IV)

dans laquelle $R^1$, $R^2$, $R^3$ et n ont les significations indiquées ci-dessus et ou bien $R^{33}$ représente l'hydrogène, un groupe alkyle inférieur, alcényle inférieur ou cycloalkylméthyle inférieur et $R^4$ représente un groupe formyle, alcanoyle inférieur ou alcoxycarbonyle inférieur, ou bien $R^{33}$ représente un groupe alkyle inférieur et $R^4$ un groupe cycloalkylcarbonyle inférieur, ou bien

c) on fait réagir un composé de formule générale

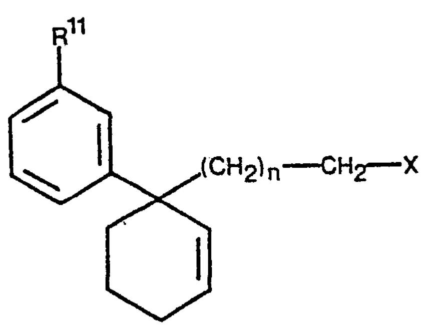

(V)

dans laquelle $R^{11}$ et n ont les significations indiquées ci-dessus et X représente un groupe éliminable, avec une amine de formule générale

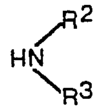

(VI)

dans laquelle $R^2$ et $R^3$ ont les significations indiquées ci-dessus, ou bien

d) on chauffe une base d'ammonium quaternaire de formule générale

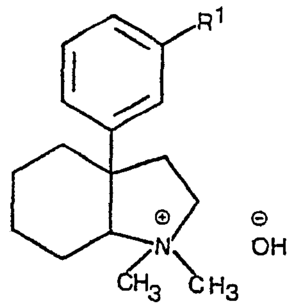

(VII)

dans laquelle $R^1$ a la signification indiquée ci-dessus, ou bien on chauffe un sel d'ammonium quaternaire correspondant approprié ou on le traite par une base, ou bien

e) dans un composé de formule générale

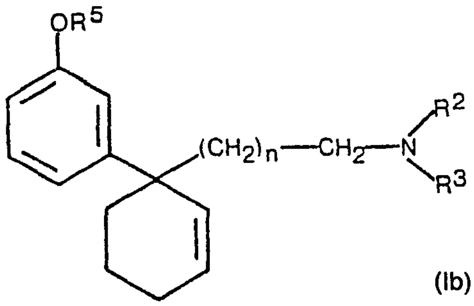

(Ib) ou

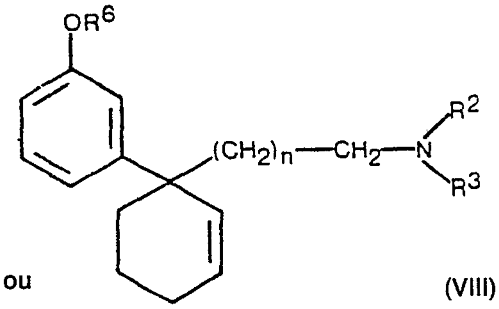

(VIII)

dans laquelle $R^2$, $R^3$ et n ont les significations indiquées ci-dessus et $R^5$ représente un groupe alkyle inférieur et $R^6$ un groupe protecteur, on élimine respectivement le groupe alkyle $R^5$ ou le groupe protecteur $R^6$, ou bien

f) on éthérifie un composé de formule générale

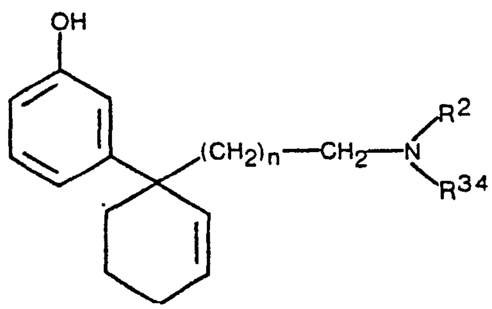

(Ic)

dans laquelle n et $R^2$ ont les significations indiquées ci-dessus et $R^{34}$ représente un groupe alkyle inférieur, alcényle inférieur ou cycloalkylméthyle inférieur, ou bien

g) on fait réagir un composé de formule générale

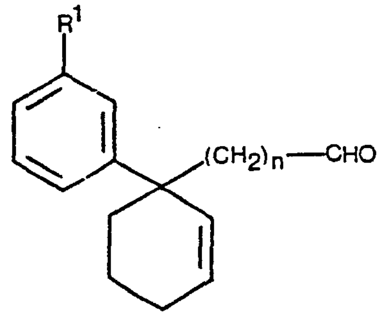

(IX)

dans laquelle $R^1$ et n ont les significations indiquées ci-dessus, dans des conditions réductrices, avec une amine de formule générale VI ci-dessus, ou bien

h) on résout un composé racémique de formule générale I en les antipodes optiques, ou bien

i) on convertit un composé de formule générale I en sel formé par addition avec un acide acceptable pour l'usage pharmaceutique.

2. Procédé selon la revendication 1, caractérisé en ce que $R^1$ représente un groupe hydroxy ou méthoxy, $R^2$ et $R^3$ représentent chacun un groupe alkyle inférieur et n est égal à 1.

3. Procédé selon la revendication 1 ou 2, caractérisé en ce que $R^2$ et $R^3$ représentent tous deux des groupes méthyle.

4. Procédé selon la revendication 3, caractérisé en ce que l'on prépare la 1-(m-méthoxy-phényl)-N,N-diméthyl-2-cyclohexène-1-éthylamine.

5. Procédé selon la revendication 3, caractérisé en ce que l'on prépare la 1-(m-hydroxyphényl)-N,N-diméthyl-2-cyclohexène-1-éthylamine.